# EUROPEAN PATENT APPLICATION

(11) **EP 1 359 160 A1**
(43) Date of publication of application: **05.11.2003**
(21) Application number: 03250919.2
(22) Date of filing: 14.02.2003
(51) Int. Cl.: C07K 14/72, G01N 33/50

(54) **Androgen receptor overexpressing skeletal myoblasts**

(30) Priority: 28.02.2002 US 360655 P
(71) Applicant: Pfizer Products Inc., Groton, Connecticut 06340 (US)
(72) Inventor: Pan, Lydia Codetta, Pfizer Global R & D, Groton, Connecticut 06340 (US); Wang, Xiao-Ning, Pfizer Global R & D, Groton, Connecticut 06340 (US)
(74) Representative: England, Peter Michael

(57) **Abstract**

This invention relates to mammalian skeletal myoblasts that overexpress the androgen receptor and screening methods for identifying and characterizing androgen receptor modulators.

## Description

### FIELD OF THE INVENTION

This invention relates to mammalian skeletal myoblasts that overexpress the androgen receptor and uses of such myoblasts in identifying and characterizing androgen receptor modulators.

### BACKGROUND OF THE INVENTION

The present invention relates generally to mammalian skeletal myoblast that overexpress the androgen receptor, and to screening methods for identifying and characterizing androgen receptor modulators.

The mammalian androgen receptor is a member of the nuclear hormone receptor superfamily. Androgens, such as testosterone and its active metabolite dihydrotestosterone (DHT), bind with high affinity to the androgen receptor. In animal models, it has been described that a testosterone deficiency due to orchidectomy results in the loss of bone from the male skeleton and that the administration of exogenous androgens can either prevent this loss or restore the lost bone mass. Hofbauer and Khosla (1999).

In humans, bone mass and levels of circulating androgens decrease with age. A correlation has been reported between osteoporosis and decreased testosterone levels. Jackson (1993). Studies have also suggested that androgen supplementation of the elderly can have beneficial effects with respect to the skeleton. Hofbauer, et al. (2000).

U.S. Patent No. 5,614,620 discloses a 3715 base pair nucleotide sequence and deduced sequences of 734 and 918 amino acid residues for human androgen receptor. U.S. Patent No. 5,614,620 also discloses the expression of the cloned human androgen receptor gene in eukaryotic and prokaryotic cells. The human androgen receptor cDNA sequence is also disclosed in Genbank Accession No. M34233 and in Genbank Accession No. M23263.

International Patent Application Publication No. WO 02/00716 discloses C2C12 mouse skeletal muscle cells having the rat androgen receptor stably introduced therein and uses of those cells by means of functional transactivation assays in assessing the efficacy of compounds as androgen receptor modulators.

There is a need for fast, inexpensive and reliable methods for identifying and characterizing androgen receptor modulators.

### SUMMARY OF THE INVENTION

One aspect of this invention provides mammalian skeletal myoblasts that overexpress an androgen receptor, preferably a human androgen receptor.

Another aspect of this invention provides transformed mammalian skeletal myoblasts comprising an inserted polynucleotide comprising a polynucleotide sequence that encodes the human androgen receptor, or a progeny cell thereof comprising said polynucleotide sequence.

In a preferred embodiment of the myoblast aspects of this invention said androgen receptor is a polypeptide sequence that is encoded by the polynucleotide sequence that hybridizes under high stringency conditions to a polynucleotide sequence selected from SEQ ID NO: 1 and SEQ ID NO: 2. In a more preferred embodiment said androgen receptor is a polypeptide sequence that is encoded by the polynucleotide sequence selected from SEQ ID NO: 1 and SEQ ID NO: 2. In an even more preferred embodiment, said androgen receptor is a polypeptide sequence selected from SEQ ID NO: 3 and SEQ ID NO: 4.

In a preferred embodiment of the transformed mammalian skeletal myoblast aspects of this invention, said polynucleotide sequence hybridizes under high stringency conditions to a polynucleotide sequence selected from SEQ ID NO: 1 and SEQ ID NO: 2. In a more preferred embodiment said polynucleotide sequence is selected from SEQ ID NO: 1 and SEQ ID NO: 2.

In a further preferred embodiment of the myoblast aspects of this invention, said myoblast is a murine skeletal myoblast. In more preferred embodiment, said myoblast is derived from a C2 murine skeletal myoblast line.

An additional aspect of this invention provides a C2hAR57 cell.

Another aspect of this invention provides screening methods comprising:
treating a cell selected from a cell of any one of claim 1-10, with an agent that modulates the androgen receptor; and
determining the effect of said agent on said cell.

A further aspect of this invention provides a screening methods comprising:
treating a cell of this invention, with an agent that modulates the androgen receptor; and
determining the effect of said agent on:
   the continued proliferation of said cell;
   the differentiation of said cell;
   the level of thymidine uptake of said cell;
   whether the cell is in G0 or G1 cell cycle phase;
   whether the cell is in G2-M or S cell cycle phase;
   the formation of myotubes of said cell;
   the level of protein content of said cell;
   the level of IGF-II expression of said cell; or
   the level of IGFBP4 expression of said cell.

Another aspect of this invention provides screening methods for identifying an androgen receptor agonist comprising:
treating a cell of this invention with a test agent;
determining the effect of said agent on the proliferation of said cell; and
characterizing the test agent as one of the following:
   an androgen receptor agonist, provided the agent inhibits or terminates proliferation of said cell; or
   an agent that is not androgen receptor agonist, provided the agent does not inhibit or terminate proliferation of said cell.

In a preferred embodiment of the screening method aspects of this invention comprising determining the effect of said agent on the proliferation of said cell, the inhibition or termination of proliferation is determined by a method comprising measuring thymidine uptake of said cell. In another preferred embodiment, the inhibition or termination of proliferation is determined by a method comprising determining whether the cell is in G0 or G1 cell cycle phase. In a further preferred embodiment, the inhibition or termination of proliferation is determined by a method comprising determining whether the cell is in G2-M or S cell cycle phase.

An additional aspect of this invention provides screening methods for identifying an androgen receptor agonist comprising:
treating a cell of this invention with a test agent;
determining the effect of said agent on the differentiation of said cell; and
characterizing the test agent as one of the following:
   an androgen receptor agonist, provided the agent promotes the differentiation of said cell; or
   an agent that is not an androgen receptor agonist, provided the agent does not promote the differentiation of said cell.

In a preferred embodiment of the screening method aspects of this invention comprising determining the effect of said agent on differentiation of said cell, the promotion of differentiation of said cell is determined by a method comprising determining the formation of myotubes of said cell. In another preferred embodiment, the promotion of differentiation of said cell is determined by a method comprising measuring the level of protein content of said cell. In an additional preferred embodiment, the promotion of differentiation of said cell is determined by a method comprising measuring the level of IGF-ll expression of said cell. In a further preferred embodiment, the promotion of differentiation of said cell is determined by a method comprising measuring the level of IGFBP4 expression of said cell.

A further aspect of this invention provides screening methods for identifying an androgen receptor agonist comprising:
treating a cell of this invention with a test agent and mitogen;
withdrawing said mitogen;
determining the effect of said agent on apoptosis as a result of said mitogen withdrawal; and
characterizing the test agent as one of the following:
   an androgen receptor agonist, provided the agent inhibits or prevents apoptosis of said cell; or
   an agent that is not an androgen receptor agonist, provided the agent does not inhibit or prevent apoptosis of said cell.

Another aspect of this invention provides screening methods for identifying an androgen receptor antagonist comprising:
treating a cell of this invention with an androgen receptor agonist and a test agent;
determining the effect of said agent on:
   the continued proliferation of said cell;
   the differentiation of said cell;
   the level of thymidine uptake of said cell;
   whether the cell is in G0 or G1 cell cycle phase;
   whether the cell is in G2-M or S cell cycle phase;
   the formation of myotubes of said cell;
   the level of protein content of said cell;
   the level of IGF-II expression of said cell; or
   the level of IGFBP4 expression of said cell.

An additional aspect of this invention provides screening methods for identifying an androgen receptor antagonist comprising:
treating a cell of this invention with mitogen, an androgen receptor agonist and a test agent;
withdrawing said mitogen determining the effect of said agent on apoptosis of the cell.

In a preferred embodiment of the screening methods of the invention for identifying and/or characterizing an androgen receptor agonist, a cell of this invention is treated with a test agent in an amount sufficient to agonize the androgen receptor.

In a preferred embodiment of the screening methods of the invention for identifying and/or characterizing an androgen receptor antagonist, a cell of this invention is treated with an androgen receptor agonist in an amount sufficient to agonize the androgen receptor and said cell is treated with a test agent in an amount sufficient to antagonize the androgen receptor.

In a preferred embodiment of the screening methods of the invention wherein a cell of the invention is treated with a mitogen, the cell is treated with said mitogen in an amount sufficient to stimulate mitosis or cell proliferation.

In a preferred embodiment of the screening methods of the invention wherein a cell of the invention is treated with a mitogen and then the mitogen is withdrawn, said mitogen is withdrawn by an amount sufficient to reduce the rate of mitosis or proliferation of said cell.

A further aspect of this invention provides methods of treating a condition or disease characterized by a loss of bone mass, comprising administering an androgen receptor agonizing amount of an agent identified by a screening method of this invention to a subject, preferably a human, in need thereof.

A further aspect of this invention provides methods of treating a condition or disease characterized by a loss of muscle mass or muscle wasting, comprising administering an androgen receptor agonizing amount of an agent identified by a screening method of this invention to a subject, preferably a human, in need thereof.

Unless defined otherwise, all technical and scientific terms used herein have the same meanings as commonly understood by one of ordinary skill in the art to which this invention belongs. Although any machines, materials, and methods similar or equivalent to those described herein can be used to practice or test the present invention, the preferred machines, materials and methods are now described. All publications mentioned herein are cited for the purpose of describing and disclosing the cell lines, protocols, reagents and vectors which are reported in the publications and which might be used in connection with the invention. Nothing herein is to be construed as an admission that the invention is not entitled to antedate such disclosure by virtue of prior invention.

"Agonist" refers to an agent which activates the androgen receptor or which intensifies the biological activity of the androgen receptor. The term "androgen receptor agonist" is used synonymously with the term "androgen". The verb to "agonize" refers to the activation of the androgen receptor or the intensification of the biological activity of the androgen receptor by an agonist. More preferably, an agonist is an agent that, at a concentration of 10,000 nM or less, activates the androgen receptor by at least about 1%, more preferably about 5%, even more preferably about 10% and especially more preferably about 25% of the maximally efficacious dose of DHT. Agonists may include proteins, nucleic acids, carbohydrates, small molecules or any other agents which modulate the activity of the androgen receptor either by directly interacting with the androgen receptor or by acting on components of the biological pathway in which the androgen receptor participates. The maximally efficacious dose of DHT is one that, if exceeded, fails to increase the magnitude of the observed response. Those skilled in the art will appreciate that the maximally efficacious dose will be dependent upon a number of factors, including the method used for measuring a response and, if applicable, the type of cell and/or level of androgen receptor expression used to measure the response.

"Antagonist" refers to an agent which inhibits or attenuates the biological activity of the androgen receptor of mammalian skeletal muscle cells. Preferably, an antagonist is an agent that, at a concentration of 10,000 nM or less, inhibits or attentuates the biological activity of the androgen receptor of mammalian skeletal muscle cells exposed a maximally efficacious dose of DHT by at least about 1%, more preferably about 5%, even more preferably about 10% and especially more preferably about 25%. The verb to "antagonize" refers to the inhibition or the attenuation of the biological activity of the androgen receptor of mammalian skeletal muscle cells by an antagonist. Antagonists may include proteins, nucleic acids, carbohydrates, small molecules or any other agents which modulate the activity of the androgen receptor either by directly interacting with the androgen receptor or by acting on components of the biological pathway in which the androgen receptor participates. Antagonists may also include agents which block or prevent agonists from modulating the activity of the androgen receptor.

"Hybridization" refers to the process by which a polynucleotide strand anneals with a complementary strand through base pairing under defined hybridization conditions. Specific hybridization is an indication that two nucleic acid sequences share a high degree of identity. Specific hybridization complexes form under permissive annealing conditions and remain hybridized after the "washing" step(s). The washing step(s) is particularly important in determining the stringency of the hybridization process, with more stringent conditions allowing less non-specific binding, i.e., binding between pairs of nucleic acid strands that are not perfectly matched. Permissive conditions for annealing of nucleic acid sequences are routinely determinable by one of ordinary skill in the art and may be consistent among hybridization experiments, whereas wash conditions may be varied among experiments to achieve the desired stringency, and therefore hybridization specificity. Permissive annealing conditions occur, for example, at 68°C in the presence of about 6 x SSC, about 1% (w/v) SDS, and about 100 µg/ml denatured salmon sperm DNA.

Generally, stringency of hybridization is expressed, in part, with reference to the temperature under which the wash step is carried out. Generally, such wash temperatures are selected to be about 5°C to 20°C lower than the thermal melting point (Tₘ) for the specific sequence at a defined ionic strength and pH. The Tₘ is the temperature (under defined ionic strength and pH) at which 50% of the target sequence hybridizes to a perfectly matched probe. An equation for calculating Tₘ and conditions for nucleic acid hybridization are well known and can be found in Sambrook et al., 1989, Molecular Cloning: A Laboratory Manual, 2^{nd} ed., vol. 1-3, Cold Spring Harbor Press, Plainview, NY; specifically see volume 2, chapter 9.

"High stringency conditions" refers to hybridization between polynucleotides of the present invention which include wash conditions of 68°C in the presence of about 0.2 x SSC and about 0.1% SDS, for 1 hour. Alternatively, temperatures of about 65°C, 60°C, 55°C, or 42°C may be used. SSC concentration may be varied from about 0.1 to 2 x SSC, with SDS being present at about 0.1%. Typically, blocking reagents are used to block non-specific hybridization. Such blocking reagents include, for instance, denatured salmon sperm DNA at about 100-200 µg/ml. Organic solvent, such as formamide at a concentration of about 35-50% v/v, may also be used under particular circumstances, such as for RNA:DNA hybridizations. Useful variations on these wash conditions will be readily apparent to those of ordinary skill in the art. Hybridization, particularly under high stringency conditions, may be suggestive of evolutionary similarity between the nucleotides. Such similarity is strongly indicative of a similar role for the nucleotides and their encoded polypeptides.

"Modulator" refers to an agent that is either an androgen receptor agonist or an androgen receptor antagonist.

"Mitogen" refers to an agent that stimulates mitosis or cell proliferation of myoblasts. For example, fetal calf serum, basic fibroblast growth factor and epidermal growth factor (also known as TGF-α) are mitogens that stimulate mitosis or cell proliferation of myoblasts.

"Myoblast" means a mononucleated, undifferentiated cell capable of giving rise to one or more differentiated muscle cells including myocytes or myotubes (i.e., myogenesis), and includes such cells as they occur naturally or as they may be altered by natural or artificial mutation, DNA recombination, transformation or the like.

"Overexpress" and "overexpression" refer to a mammalian skeletal myoblast or myoblast cell line that expresses the androgen receptor at a level that is higher than the level required to specifically bind at least about 2.2 femtomoles DHT per milligram protein, preferably at least about 5 femtomoles DHT per milligram protein and more preferably at least about 10 femtomoles DHT per milligram protein, wherein the myoblast or myoblast cell line is viable and exhibits myogenic function when exposed to an androgen receptor agonist. Myogenic function is exemplified by the formation of differentiated myotubes when, for example, a myoblast is switched from a high mitogen growth medium to a low mitogen fusion medium. A suitable preparation of high mitogen growth medium and low mitogen fusion medium is described below in the Detailed Description and Examples.

"Specific binding" and "specifically binding" refer to the interaction between a protein or peptide and an agonist, an antibody, an antagonist, a small molecule, or any natural or synthetic binding composition. The interaction is dependent upon the presence of a particular structure of the protein, e.g., the antigenic determinant or epitope, recognized by the binding molecule. For example, if an antibody is specific for epitope "A," the presence of a polypeptide containing the epitope A, or the presence of free unlabeled A, in a reaction containing free labeled A and the antibody will reduce the amount of labeled A that binds to the antibody.

"Transformation" describes a process by which exogenous DNA enters and changes a recipient cell. Transformation may occur under natural or artificial conditions according to various methods well known in the art, and may utilize on any such known method for the insertion of foreign nucleic acid sequences into a prokaryotic or eukaryotic host cell, as the case may be. The method for transformation may be selected, for example, based on the type of host cell being transformed and may include, but is not limited to, viral infection, electroporation, heat shock, lipofection, and particle bombardment. "Transformed" cells includes stably transformed cells in which the inserted DNA is capable of replication either as an autonomously replicating plasmid or as part of the host chromosome, as well as transiently transformed cells which express the inserted DNA or RNA for limited periods of time.

### BRIEF DESCRIPTION OF THE DRAWINGS

FIGURE 1 shows the specificity of [³H]-DHT to cell extracts from C2hAR57 cells.
FIGURES 2B and 2D are immunofluorescence micrographs of C2C12 and C2hAR57 cells, respectively, showing the degree of binding of rabbit anti-androgen receptor antisera (PA1-111) as a primary antibody and Cy-3-conjugated anti-rabbit immunoglobulin as a secondary antibody.
FIGURE 2F is an immunofluorescence micrograph of C2hAR57 cells treated with the secondary Cy-3 anti-rabbit immunoglobulin only.

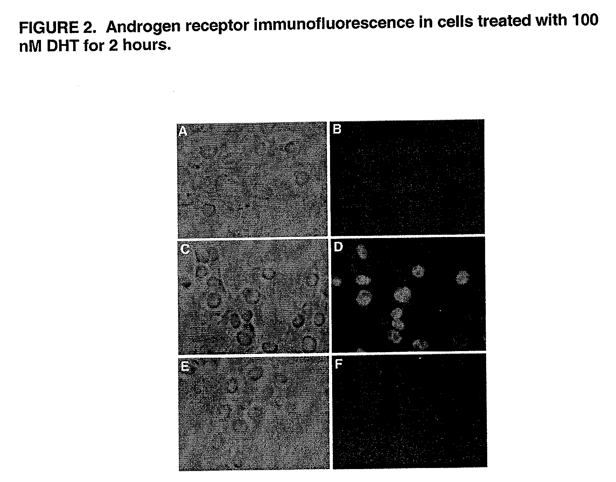

FIGURES 2A, 2C and 2E are phase-contrast illuminated micrographs corresponding to 2B, 2D and 2F, respectively.

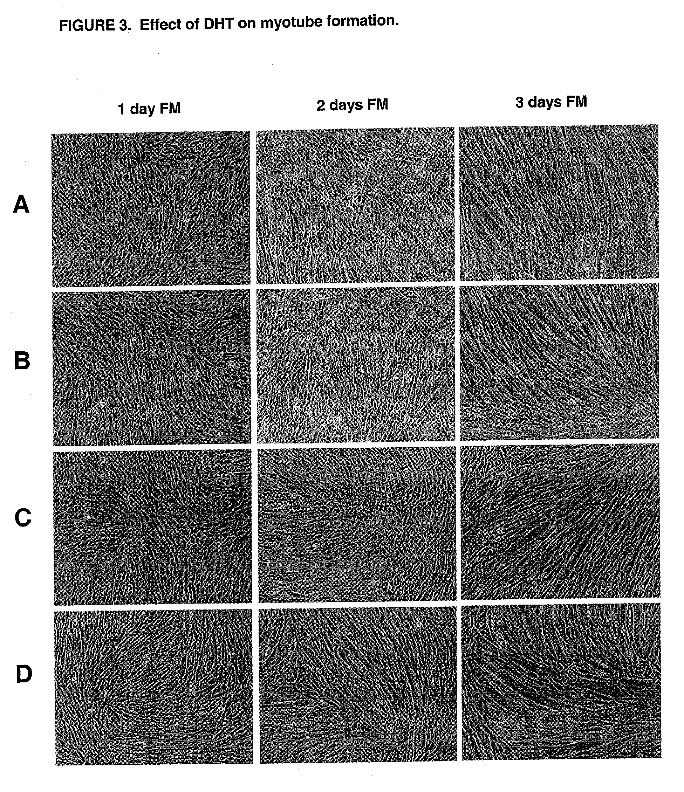

FIGURE 3 shows the effect of DHT on myotube formation in C2C12 and C2hAR57 cells.

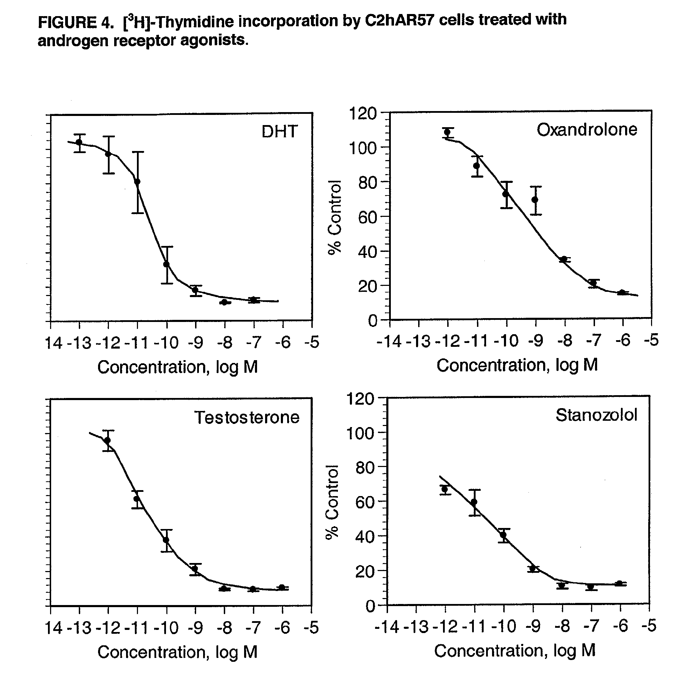

FIGURE 4 shows the effect of four androgen receptor agonists on [³H]-thymidine incorporation in C2hAR57 cells.

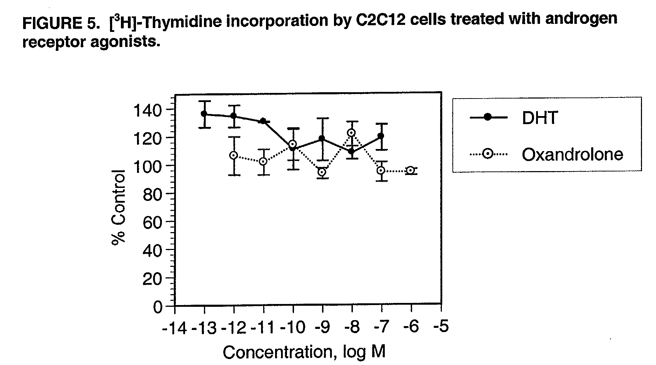

FIGURE 5 shows the effect of two androgen receptor agonists on [³H]-thymidine incorporation in C2C12 cells.

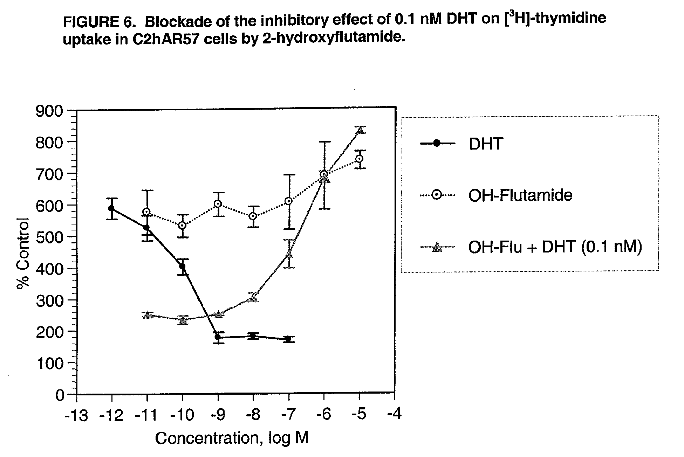

FIGURE 6 shows the effect of the androgen receptor antagonist, 2-hydroxyflutamide, on [³H]-thymidine incorporation in C2hAR57 cells.

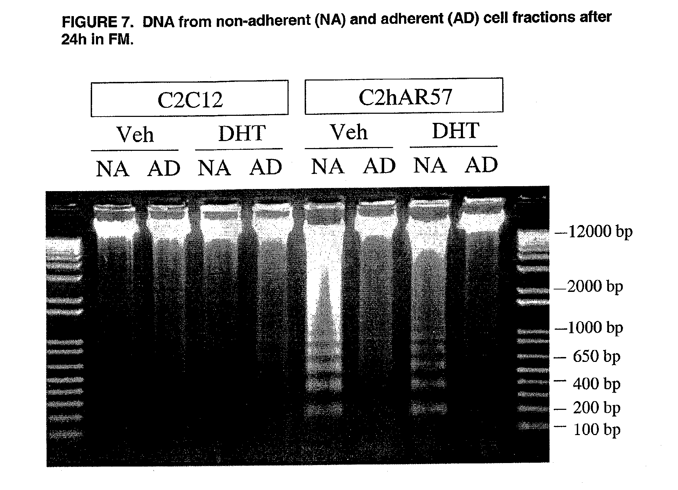

FIGURE 7 shows the effect of DHT on cell apoptosis in C2C12 and C2hAR57 cells.

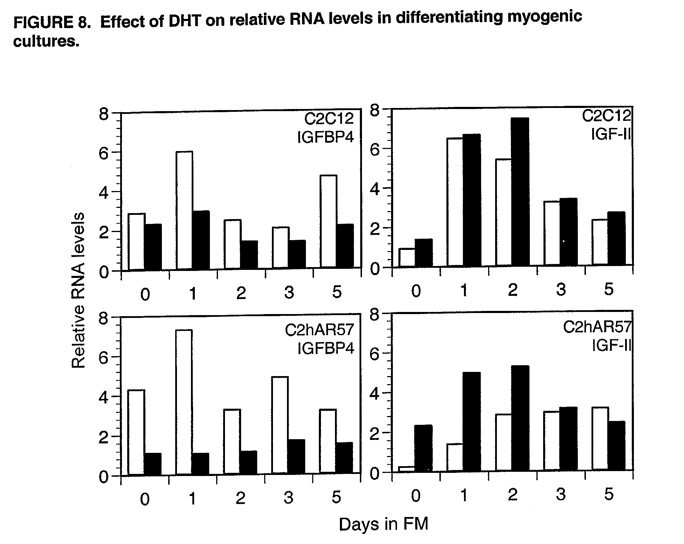

FIGURE 8 show the effect of DHT on IGF-ll and IGFBP4 expression in C2C12 and C2hAR57 cells based upon Northern blot analyses.

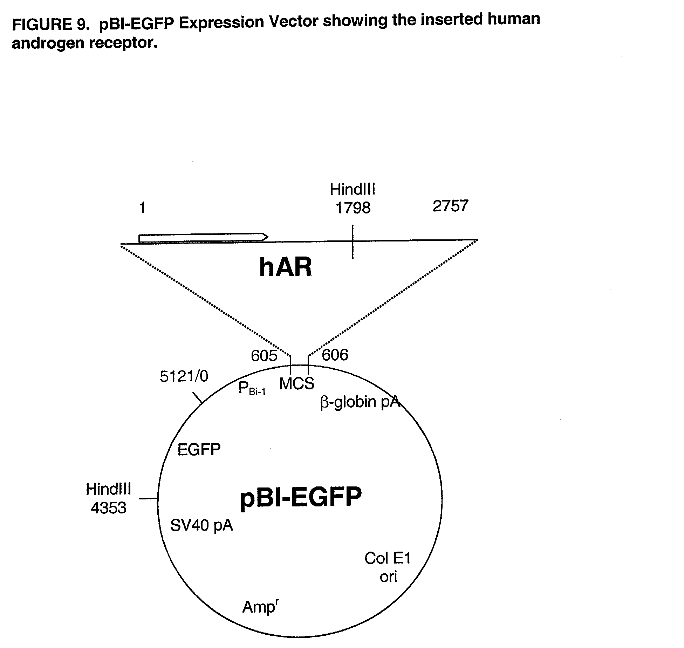

FIGURE 9 is a map of the pBl-EGFP expression vector showing the inserted human androgen receptor.

### DETAILED DESCRIPTION OF THE INVENTION

The present invention relates, in part, to mammalian skeletal myoblastic cells that overexpress the androgen receptor. To express a biologically active androgen receptor, a suitable nucleotide sequences encoding the androgen receptor may be inserted into an appropriate expression vector. Based upon the present disclosure, those with skill in the art may use any suitable known method to construct expression vectors containing sequences encoding the androgen receptor and appropriate transcriptional and translational control elements. These methods include, for example, *in vitro* recombinant DNA techniques, synthetic techniques, and in vivo genetic recombination (*see, e.g*., Sambrook, J. et al. (1989); Ausubel, F.M. et al. (2001)).

The elements for transcriptional and translational control in a suitable host of the inserted coding sequence of the androgen receptor may include regulatory sequences, such as enhancers, constitutive and inducible promoters, and 5' and 3' untranslated regions in the vector and in polynucleotide sequences encoding the androgen receptor. As those skilled in the art will appreciate, such elements vary in their strength and specificity. Specific initiation signals may also be used to achieve more efficient translation of sequences encoding the androgen receptor. Such signals include the ATG initiation codon and adjacent sequences, e.g. the Kozak sequence. For the purpose of carrying out the invention where sequences encoding the androgen receptor and its initiation codon and upstream regulatory sequences are inserted into the appropriate expression vector, no additional transcriptional or translational control signals may be needed. However, where only coding sequence, or a fragment thereof, is inserted, exogenous translational control signals including an in-frame ATG initiation codon should be provided by the vector. Exogenous translational elements and initiation codons may be of various origins, both natural and synthetic. The efficiency of expression may be enhanced by the inclusion of enhancers appropriate for the particular host cell system used *(see,* e.g., Scharf, D. et al. (1994)).

Preferably, an expression vector used in the preparation of the cells of this invention comprises a polynucleotide encoding the androgen receptor that is operatively linked to a promoter region and/or a promoter region that is operatively linked to an enhancer region (i.e., an enhancer-promoter). Promoters and enhancer-promoters are regions of a DNA molecule that can drive expression of a target peptide in a host cell. The pBl-EGFP Tet vector (Clontech, Cat. #6154-1, Palo Alto, CA) contains the bidirectional promoter *P*_{bi-1} which is responsive to the tTA and rtTA regulatory proteins in the Tet-Off™ and Tet-On™ Gene Expression Systems, respectively. The Tet-On™ and Tet-Off™ systems enable the regulation of gene expression using varying concentrations of tetracycline or tetracycline derivatives.

An expression vector used in the preparation of cells of this invention may be prepared by standard recombinant DNA cloning techniques well known to those skilled in the art, based upon the present disclosure. In a preferred method for preparing such an expression vector, a DNA fragment containing the androgen receptor coding sequence, preferably the 2577-bp human androgen receptor coding sequence (Genbank Accession No M23263), is ligated into the unique Pvull site of the pBI-EGFP Tet vector, as shown in Figure 9. The resulting expression vector is then transformed and amplified in *E. coli* strain DH5α (Life Technologies, Rockville, MD)?.

It will be appreciated by those skilled in the art based upon the present disclosure, that a host mammalian skeletal myoblast strain may be used in the preparation of a cell of this invention. Any of the mammalian skeletal myoblast lines may be used in the preparation of the cells of this invention, including but not limited to L6 (Yaffe and Saxel (1977)), L6E9 (Benoff, S. and B. N. Nadal-Ginard (1979)), L8 (Richler and Yaffe (1970)), C2C12 (see below), MM14 (Lim and Hauschka (1984)), G7 (Christian, C. N., et al. (1977)), G8 (Christian, C. N., et al. (1977)), Sol8 (Daubas, P., et al. (1988)), BC3H1 (Schubert, D., et al. (1974)), H9c2 (Kimes, B. W. and B. L. Brandt (1976)), SJRH30[RMS 13] (Douglass, et al. (1987)) and P19 (McBurney, M. W., et al. (1982)). A preferred mammalian skeletal myoblast for use in the preparation of a cell of this invention is from the murine muscle-derived cell line, C2C12, described in Yaffe and Saxel (1977) and Blau, H. M., G. K. Pavlath, et al. (1985) Science 230(4727), 758-766 (available from the American Type Tissue Culture collection (ATCC), ATCC No. CRL-1772, Manassas, VA, contributed by B. Paterson).

The preparation and utilization of mammalian skeletal myoblast lines may be carried out based upon methods known in the art, based upon the present description. For example, sequences encoding the androgen receptor can be transformed into cell lines using an expression vector prepared by the preferred method described above and having a selectable marker gene on the same or on a separate vector. The method for transformation may include, but is not limited to, viral infection, electroporation, heat shock, lipofection and particle bombardment. For the purpose of generating a cell line that overexpresses the androgen receptor, it is preferred that sequential transfection with multiple DNA molecules be employed, for example using Lipofectamine^{TM} reagents (Life Technologies).

Following the introduction of the vector, cells may be allowed to grow for a suitable period of time, such as, for example, about one to two days in enriched media before being switched to selective media. The purpose of the selectable marker is to confer resistance to a selective agent, and its presence allows growth and recovery of cells which successfully express the introduced sequence. As those with skill in the art will appreciate, based upon the present description, resistant clones of stably transformed cells may be propagated using tissue culture techniques appropriate to the cell type.

It will further be appreciated by those with skill in the art, based upon the present disclosure, that any suitable selection systems ("selectivity markers") may be used to recover transformed cell lines. These include, but are not limited to, the herpes simplex virus thymidine kinase and adenine phosphoribosyltransferase genes, for use in tk- and apr- cells, respectively (*see*, e.g., Wigler, M. et al. (1977); Lowy, I. et al. (1980) *Cell* 22, 817-823). Antimetabolite, antibiotic, or herbicide resistance can also be used as a basis for selection. For example, *dhfr* (dihydrofolate reductase) confers resistance to methotrexate; *neo* confers resistance to the aminoglycosides neomycin and G-418; and *als* and *pat* confer resistance to chlorsulfuron and phosphinotricin acetyltransferase, respectively (*see*, Wigler, M. et al. (1980); Colbere-Garapin, F. et al. (1981)). Additional selectable genes have been described, e.g., *trpB* and *hisD,* which alter cellular requirements for metabolites (*see*, e.g., Hartman, S.C., et al. (1988)). Visible selectivity markers, e.g., anthocyanins, green fluorescent proteins (Clontech, Palo Alto, CA), β glucuronidase and its substrate β-glucuronide, or a luciferase and its substrate luciferin may be used. These markers can be used not only to identify transformants, but also to quantify the amount of transient or stable protein expression attributable to a specific vector system *(see,* e.g., Rhodes, C.A. (1995)).

Although the presence or absence of selectivity marker gene expression suggests, as the case may be, that the gene of interest is also present, it may be desirable, in any such case, to confirm the presence and expression of the gene. For example, if the sequence encoding the androgen receptor is inserted within a selectivity marker gene sequence, transformed mammalian skeletal myoblasts containing sequences encoding the androgen receptor can be identified by the absence of selectivity marker gene function. Alternatively, a selectivity marker gene can be placed in tandem with a sequence encoding the androgen receptor under the control of a single promoter. As those skilled in the art will appreciate, based upon the present disclosure, expression of the marker gene in response to induction or selection generally indicates expression of the tandem gene as well.

Host mammalian skeletal myoblasts that contain a nucleotide sequence encoding the androgen receptor and that overexpress the androgen receptor polypeptide may be identified by a variety of procedures known to those of skill in the art based upon the present disclosure. These procedures include, but are not limited to, DNA-DNA or DNA-RNA hybridizations, polymerase chain reaction (PCR) amplification, and protein bioassay or immunoassay techniques which include membrane-, solution- and/or chip-based technologies for the detection and/or quantification of nucleotide or amino acid sequences. Such methods and techniques are described, for example, in Ausubel, F.M. et al. (2001).

A preferred method to confirm overexpression of the androgen receptor is through the use of a [³H]-DHT competition binding assay, for example, as described in Liao, S., et al. (1984). Immunological methods for detecting and measuring the expression of androgen receptor are also preferred methods for use in carrying out the invention. For example, such methods include using specific polyclonal or monoclonal antibodies. Such methods are well known in the art. Examples of such techniques include enzyme-linked immunosorbent assays (ELISAs), radioimmunoassays (RIAs), and fluorescence activated cell sorting (FACS). These and other assays are well known in the art (*see*, e.g., Hampton, R. et al. (1990); Coligan, J.E. et al. (1997); and Pound, J.D. (1998).

Any mammalian androgen receptor may be used in the preparation of the myoblasts of this invention. Androgen receptors useful in the present invention may be isolated from various mammalian species. Rat androgen receptors are described in Genbank Accession No. M23264 and JO5454, as well as by Chang et al. (1988)), 324-326. Mouse androgen receptors are set forth in Genbank Accession No. M37890 and by Gaspar et al. (1991) and He et al. (1990). A hamster androgen receptor is described by Shiba et al. (2001). The dog androgen receptor is described in Lu, B., S. Smock, et al. (2001). The human androgen receptor is described in Genbank Accession No. M34233 and Genbank Accession No. M23263.

The mammalian androgen receptor used in the preparation of the myoblasts of this invention may vary with respect to the length or identity of the amino acid sequence or the nucleotide sequence encoding it, so long as the androgen receptor is expressed in a cell of this invention and biologically functions in a cell of this invention when exposed to an androgen receptor modulator in a substantially similar manner as the human androgen receptor described in Genbank Accession No. M23263.

In a preferred embodiment of the present invention, the myoblasts of this invention are prepared according to methods known to those skilled in the art, based upon the present disclosure, using the murine muscle-derived cell line, C2C12 (ATCC No. CRL-1772) as host cell transformed according to methods known to those skilled in the art, based upon the present disclosure, using an expression vector containing the androgen receptor coding sequence, preferably the human androgen receptor coding sequence (Genbank Accession No. M23263), resulting in a mammalian skeletal myoblast that overexpresses the androgen receptor. A preferred cell of this invention is a cell of the cell line designated C2hAR57.

The cells of this invention are particularly useful for the identification of agents that are modulators of the androgen receptor. Methods used for such identification are based upon detecting changes in such cells, including the level and/or rate of proliferation of the cells, the level and/or rate of differentiation of the cells, and/or the level and/or rate of apoptosis of the cells. Therefore, it is particularly useful to maintain the cells in a proliferating and undifferentiated state, preferably by propagating such cells in a high mitogen growth medium of Dulbecco's Modified Eagle's Medium (Life Technologies) supplemented with 15% fetal bovine serum (Gemini Bio-Products, Calabasas, CA) and 10 pM basic fibroblast growth factor (Collaborative Biomedical Products, Bedford, MA).

In one embodiment of the screening methods of this invention, the effect on the rate of proliferation of cells of this invention may be used to identify and/or characterize a potential androgen receptor modulator ("test agent"). This embodiment is based upon the observation that the cells of this invention in the presence of an androgen receptor agonist decrease their level of proliferation. In a preferred embodiment, the uptake of thymidine, preferably radio-labeled thymidine, more preferably [³H]-thymidine, is used to identify and characterize an androgen receptor agonist. The method comprises exposing actively proliferating skeletal myoblasts that overexpress the androgen receptor of this invention to a test agent in growth medium for a suitable period of time, such as for example, about 48 hours, followed by exposure to radio-labeled thymidine (preferably [³H]-thymidine, Amersham, Buckinghamshire, UK) in culture medium for a suitable period of time, such as for example, about two hours. Thymidine incorporation is terminated by removal of the culture medium followed by washing with ice-cold phosphate buffered saline followed by 5% trichloroacetic acid. The cells are then lysed. The radioactivity of the lysate is then measure to determine the degree of radio-labeled thymidine that has been incorporated into the cell. A test agent would test positive as an androgen receptor agonist if it causes a detectable decrease in the rate of thymidine incorporation as compared to a control sample containing no androgen receptor androgen receptor agonist. Preferably, such decrease is at least about 30% below that of the control, and more preferably, at least about 50% below that of the control.

In another embodiment of the screening methods of this invention, the methods for measuring uptake of radio-labeled thymidine in cells of this invention, as described above, may also be used to characterize an androgen receptor modulator. This is based upon the observation that the degree by which an androgen receptor agonist inhibits [³H]-thymidine uptake is consistent with the agonist's known affinity for the androgen receptor. This effect has been shown for the natural androgen receptor agonists, testosterone and DHT, as well as for synthetic androgen receptor agonists, such as oxandrolone and stanozolol, as shown in FIGURE 4. An androgen receptor agonist may be tested using the thymidine uptake method described above and the results may be compared to the known effect of other androgen receptor agonists. The results may be used to predict the relative affinity to the androgen receptor of the test agent as compared to known androgen receptor agonists.

In another embodiment of the screening methods of this invention, increases in the proportion of cells in G0 and G1 phase of the cell cycle and/or a concomitant decrease in the fraction of cells in the replicative (S) and post-replicative (G2/M) phases are used to assess the inhibition of cell proliferation by a test agent. In G0/G1, the amount of cellular DNA corresponds to a diploid content of chromosomes (2N). During S phase, chromosomal replication increases DNA content from 2N to 4N, and the chromosomal content of cells in G2/M (prior to cell division) is 4N.

A preferred quantitative DNA stain is propidium iodide (PI; Molecular Probes, product no. P-3566, Calbiochem; La Jolla, Calif.). Other useful quantitative DNA stains include fluorescent nucleic acid stains. As those skilled in the art will appreciate, based upon the present disclosure, a variety of suitable nucleic acid stains are known in the art, including but not limited to, thiazole orange, ethidium homodimer, ethidium bromide, HOECHST 33258, and DAPI. Nucleic acid stains can be intercalating dyes, such as phenanthridines and acridines, e.g., ethidium bromide and propidium iodide; minor-groove binders, such as DAPI and the Hoechst dyes; or not belonging in either of these two categories, such as acridine orange, 7-AAD and hydroxystilbamidine. Cyanine dyes are dyes of choice based on their high molar absorptivity, very low intrinsic fluorescence, large fluorescence enhancements upon binding to nucleic acids and moderate to very high affinity for nucleic acids, with little or no staining of other biopolymers. Some nucleic acid stains are cell-impermeant and other stains are cell permeant stains. Cell permeant stains do not require permeabilization of cells prior to staining. Exemplary cell permeant dyes are the cyanine dyes (SYTO nucleic acid stains), hexidium iodide, dihydroethidium, and ethidium homodimers.

Other available dyes include the intercalators 7-amino actinomycin D and actinomycin D, multicolor hydroxystilbamidine, Long-Wavelength LDS751 and Neurtrace Fluorescent Nissl Stains. Another quantitative DNA stain is the base analog 5-bromo-2'-deoxyuridine (BrdU, available from Sigma-Aldrich, St. Louis, MO). Additional useful nucleic acid stains are described in International Patent Application Publications WO 93/06482 (issued as U.S. Patent No. 5,582,977) and WO 94/24213 (issued as U.S. Patent No. 5,436,134) and U.S. Patents 5,321,130, 5,410,030; 5,436,134; and 5,437,980.

Propidium iodide (Pl; Calbiochem) can be added to a cell population at a concentration of at least about 0.1 µg/ml to about 100 µg/ml, preferably at least about 0.1 to about 10 µg/ml, and most preferably from about 1 to about 5 µg/ml. The cells can be incubated for about 1 minute to about 2 hours, preferably for about 10 minutes to one hour, and most preferably for about 15 to about 30 minutes at room temperature, optionally in the darkness. Incubation can also be carried out on ice, for at least about 1 minute, preferably at least about 5 minutes, and most preferably for at least about 15 minutes.

Generally, cells are stained with a quantitative DNA stain in an amount and for a time sufficient to permit detection and quantification of the DNA in the cells. Suitable staining procedures for a particular cell type and detection method can be determined according to methods known in the art, e.g., by conduction of assays using several DNA stains, concentration and incubation times, such as to determine which conditions permit suitable staining of the cells.

Staining of cells for DNA content may be accompanied by treatment of the cells with an agent that prevents staining of nucleic acids other than chromosomal DNA, e.g., RNA. In an illustrative embodiment, the cells are treated with an agent that degrades RNA, e.g., an RNase. For example, RNase (BD Clontech) can be added at about 100-500 µg/ml. Generally, cells are incubated with an agent that prevents staining of nucleic acids other than chromosomal DNA in a concentration and for a time sufficient to permit predominant staining of the chromosomal DNA over staining of the other nucleic acids. Suitable conditions can be determined according to methods in the art, e.g., by conducting assays using different agents in different conditions, such as to determine suitable conditions for the method used.

A preferred method for analyzing the stained cells is by flow cytometry or laser scanning cytometry, using, multi- parameter (or color) displays. Flow cytometry can be performed with a fluorescent activated cell sorter (FACS) as further described herein and as known in the art. Exemplary FACS machines that can be used include FACSCalibur™ Flow Cytometry System flow cytometer (Becton Dickinson Immunocytometry Systems, San Jose, CA) and a Coulter flow cytometer (Hialeah, Fla., USA) EPICS Elite®. Quantification can be performed using CellQuest (Becton Dickinson; Mountain View, CA), WinList (Verity Software House, Inc. Topsham, Maine), Multicycle software (Phoenix Flow Systems, San Diego, CA) and FACScan (Becton Dickinson, Mountain View, CA) software. A flow cytometer measures the amount of light-emitting substance associated with each cell and other parameters and provides output in the form of, e.g., a histogram, dot plot, or fraction table. As fluid containing cells passes through the light source, typically one-by-one, they are exposed to light of various wavelengths. Each particle detected by the cytometer is termed an "event." The degree to which an event transmits or scatters some of the incident light provides a measure of the event's characteristics, e.g., associated light emitting substance. For example, the event may emit light of its own accord or may emit fluorescent light generated by a fluorescent substance introduced into the event. The intensity of the emitted or reflected light is measured and stored by the cytometer.

In a preferred embodiment of the screening methods of this invention, wherein increases in the proportion of cells in G0 and G1 phase of the cell cycle and/or a concomitant decrease in the fraction of cells in the replicative (S) and post-replicative (G2/M) phases are used to assess inhibition of cell proliferation, the percentage of cells at each stage of the cell cycle may be determined by flow cytometry after staining with propidium iodide according to Krishan, A. (1975). According to this method, cells are plated at low density in high mitogen growth medium and treated for about 24 hours to about 48 hours with a test compound. Cells are then harvested by trypsinization and resuspended in 70% ethanol in Dulbecco's phosphate buffered saline (Life Technologies) for at least two hours at-20°C. The cells are then pelleted using low-speed centrifugation and resuspended in phosphate buffered saline containing ribonuclease A and propidium iodide. The cells are treated with ribonuclease A and propidium iodide for about 30 minutes at room temperature. Quantification of cells in G0 and G1 stage versus those in S, G2 and M stages may be performed using a flow cytometer, such as a FACScan™ flow cytometer equipped with an argon laser (488 nm emission wavelength) and Modfit™ software (Becton Dickinson lmmunocytometry Systems).

A test agent would test positive as an androgen receptor agonist if it causes a detectable increase in proportion of cells in the G0 and G1 phases of the cell cycle and/or a decrease in the proportion of cells in the S and G2/M phases of cell cycle as compared to a control sample containing no androgen receptor agonist. Preferably, the increase of cells in G0 and G1 phases is at least about five percent over the control, and more preferably, at least about 10% over the control. Preferably, the decrease of cells in S and G2/M phases is at least about five percent over the control, and more preferably, at least about 10% over the control.

In another embodiment of the screening methods of this invention, the effect on myogenic differentiation on cells of the invention may be used to identify an androgen receptor modulator. This embodiment is based upon the observation that the cells of this invention when treated with an androgen receptor agonist, accelerate their level of cell differentiation. In a preferred embodiment, the cells of this invention are used to determine the effect of a test agent on the level and rate of differentiated myotube formation. When cells of this invention are grown in high mitogen growth medium, they appear as small adherent mononuclear cells. However, when the medium is switched to a low mitogen fusion medium of Dulbecco's Modified Eagle's Medium supplemented with 2% horse serum (Life Technologies), a large portion of the mononucleated myoblasts fuse to form elongated, multimucleated myotubes. It has been observed that when the cells of this invention are exposed to an androgen receptor agonist, such as DHT, the myotubes form more rapidly than without the androgen receptor agonist. Although skeletal myoblasts that do not overexpress the androgen receptor also form myotubes when the medium is switched to fusion medium, neither the rate nor the extent of myotube formation is visibly affected by the addition of an androgen receptor agonist such as DHT. The effect of an androgen receptor agonist on the cells of this invention is shown in FIGURE 3. In a preferred embodiment of the screening methods of this invention, a screening assay is performed according to the methods described in EXAMPLE 4. A test agent would test positive as an androgen receptor agonist if it causes a detectable increase in the rate of myotube formation as compared to a control sample containing no androgen receptor agonist.

In a further embodiment of the screening methods of this invention, the effect on total protein content is used to assess the effect of a test agent on cells of the invention. This embodiment is based upon the observation that culturing the cells of this invention in the presence of an androgen receptor agonist accelerates the accumulation of protein content in the cells. In a preferred method of performing the screening method, the cells of this invention are first grown in high mitogen growth medium for about 24 hours. The medium is then replaced with a steroid free growth medium of Dulbecco's Modified Eagle's Medium having 15% charcoal-dextran treated fetal calf serum (Gemini Bio-Products) and 10 pM basic fibrobast growth factor (Collaborative Biomedical Products). After about 38 hours, the medium is replaced with fusion medium described above containing the test agent and the cells are exposed to the agent for at least about 48 hours and then for an additional period as required to reach at least 50% confluence. The medium is then replaced with fusion medium containing the test agent for up to three days. Protein concentration of cell extracts prepared by lysis of the cells, sonication and centrifugation, may be determined by any suitable method, such as for example, the Coomassie Blue dye-binding method described in Bradford, M. (1976). In a preferred embodiment, the screening assay is performed according to the methods described in EXAMPLE 5. A test agent would test positive as an androgen receptor agonist if it causes a detectable increase in the total protein content as compared to a control sample containing no androgen receptor agonist. Preferably, such increase is at least about 20% over the control, and more preferably, at least about 50% over the control.

In an additional embodiment of the screening methods of this invention, a test agent may be assessed based upon its effect on cell death or apoptosis of the cells of this invention (see Kaufmann, S.H., Hengartner, M.O. (2001); Arends, A.J., Wyllie, A.H. (1991); and Wyllie, A.H., et al. (1980)). While not wishing to be bound by any particular theory or mechanism, it is proposed that the mechanism by which an androgen receptor agonist enhances myogenic differentiation is by reducing the incidence of programmed cell death or apoptosis following mitogen withdrawal. This embodiment is based upon the observation that, following mitogen withdrawal, cells of the invention that are treated with an androgen receptor agonist exhibit a decreased level of apoptosis as compared to untreated cells of the invention. Following mitogen withdrawal, it appears that a percentage of previously adherent cells, detach from the culture dish and undergo apoptosis. A hallmark of cells undergoing apoptosis is a pattern of DNA fragmentation that produces a characteristic laddering effect due to internucleosomal fragmentation when analyzed by gel electrophoresis. Rosl, F. (1992). Apoptosis in non-adherent cells versus adherent cells may be confirmed by electrophoresis of a DNA sample of adherent cells versus non-adherent cells (see FIGURE 7). In carrying out this embodiment of the screening methods, cells of the invention are cultured in high mitogen growth medium, described above, together with the test agent for a suitable period of time, preferably about four days. The medium is changed to fusion medium, as described above, containing the test agent, and the cells are grown for about an additional 24 hours. Non-adherent cells are collecting by repeated gentle washing and lysed. Adherent cells are collected by trypsinization and then lysed. Total DNA is isolated according to procedures well known in the art, such as in Blin, N. and.D. Stafford (1976). DNA yield may be determined by UV spectroscopy at 260 nm. The reduction in apoptosis of cells of this invention will be evident by the reduction in the proportion of DNA of non-adherent cells of the invention treated with an androgen receptor agonist as compared to the amount of DNA in adherent cells of the invention. In a preferred embodiment the methods described in EXAMPLE 7 are used. A test agent would test positive as an androgen receptor agonist if it causes a detectable decrease in the percentage of non-adherent cells as compared to a control sample containing no androgen receptor agonist.

In another embodiment of the screening methods of this invention, a test agent may be assessed based upon its effect on the expression of insulin-like growth factors (IGFs), such as IGF-I and IGF-II. IGFs are well known to play a central role in the differentiation of myoblasts to myotubes *(see* Florini, J., D. Ewton, et al. (1996); Tollefsen, S., R. Lajara, et al. (1989); and Tollefsen, S., J. Sadow, et al. (1989). In performance of the screening method, cells of this invention are maintained as proliferating myoblasts in high mitogen growth medium with a test agent for at least two days whereupon they are allowed to reach high density (>70% confluence). When the cells reach high density, they may be harvested immediately or, preferably, switched to fusion medium with the test agent for about one to two days. The cells are then harvested and total RNA is isolated using Trizol® reagent (Life Technologies) according to the standard methods recommended by the manufacturer. Samples containing 20 micrograms (based on the concentration of RNA determined by UV spectroscopy) are separated by electrophoresis on formaldehyde-containing agarose gels. The separated samples are transferred to Duralon-UV™ nylon membrane (Stratagene, La Jolla, CA) by capillary blotting in 5x SSPE and probed with rat IGF-II probe. Any mammalian IGF-11 cDNA or RNA may be used as a probe in this method. Preferably said probe Preferably said probe possesses sequence homology >90% identical to a corresponding region of mouse IGF-ll cDNA or RNA. An IGF-ll probe may using rat embryo total RNA as template and using 5'-TGTTGGTGCTTCTCATCTCTTTGG-3' as the forward primer and 5'-CACAGACTGATGGTACTACATTGC-3' as the reverse primer, followed by electrophoretic separation of the DNA fragments on a 1 % agarose gel in tris-borate-EDTA (TBE) buffer and recovery of the 562-base pair product from a gel slice using a QIAquick Gel Extraction Kit (Qiagen, Valencia, CA). A double-stranded ³²P-labeled probe may be synthesized by random primer extension according to Feinberg, A. and B. Vogelstein (1983) and Feinberg, A. P. and B. Vogelstein (1984). Probes are denatured by boiling and used at a concentration of about 1-2 x 106 dpm/ml hybridization solution of 50% formamide, 6X SSC, 1 % SDS, 15X Denhardt's solution, 0.1 mg/ml sheared salmon sperm DNA. Hybridization is performed at 42°C for at least about 24 hours and the membranes are then washed under moderate stringency conditions (i.e., 0.3X SSC/0.1% SDS at 55°C). Hybridization signals are detected and quantified by phosphorimaging using a Cyclone™ Storage Phosphor System with OptiQuant™, version 3.15 software (Packard Instrument Corporation, Meriden, CT). The expression of IGF-II in the cells treated with the test agent is then compared to IGF-II expression in an untreated control sample containing no androgen receptor agonist. A test agent would test positive as an androgen receptor agonist if it causes a detectable increase in the level of IGF-II expression as compared to the control sample. Preferably, such increase is at least about a twofold increase over the control, and more preferably, at least about a three-fold increase over the control.

In a further embodiment of the screening methods of this invention, a test agent may be assessed based upon its effect on the expression of any one of a family of proteins known as IGFBP's, preferably IGFBP4. The biological activity of IGFs is modulated by a family of high affinity binding proteins known as IGFBP's. IGFBP4 has been shown to inhibit the action of IGF-l in myoblasts (McCusker, R. and D. Clemmons (1994)). Skeletal muscle IGFBP4 RNA levels are regulated in an opposite manner from IGF-I by the androgen receptor agonist, testosterone (Urban, R., Y. Bodenburg, et al. (1986)). In performance of the screening method, cells of this invention are grown and RNA is isolated, separated and blotted in substantially the same manner as the method for measuring IGF expression. Any IGFBP may be used as a probe for the screening method. Preferably the probe is IGFBP4. A 469-bp fragment from the coding region of rat IGFBP4 may be prepared be prepared as a probe by reverse transcription polymerase chain reaction using rat skeletal muscle poly A+ RNA as template and using 5'-GGCGACGAAGCCATCCACTG-3' as a forward primer and 5'-CCCGGTGCAGCTCACTCTGG-3' as a reverse primer. Denaturing and hybridization of the probe and detection of the hybridization signal may be performed substantially in the same manner as the method for measuring IGF expression. The expression of IGFBP4 in the cells treated with the test agent is then compared to IGFBP4 expression in an untreated control sample containing no androgen receptor agonist. A test agent would test positive as an androgen receptor agonist if it causes a detectable decrease in the level of IGFBP4 expression as compared to the control sample. Preferably, such decrease is at least about 50% over the control, and more preferably, at least about 70% over the control.

Test agents to be tested and/or characterized for activity in the screening methods of this invention are preferably tested in a range of concentrations that range from about 0.001 nM to about 10,000 nM.

It will be apparent to one with skill in the art, based upon this disclosure, that the screening methods of this invention may be used for the identification and/or characterization of androgen receptor agonists as well as for the identification and/or characterization of agents that are androgen receptor antagonists. For example, any of the methods of the invention may be used with a known androgen receptor agonist to test an agent having possible androgen receptor antagonist activity. A reduction in the positive results that would be expected in performing the screening method may indicate that the unknown agent is an androgen receptor antagonist. For example, in the screening methods of this invention based upon the uptake of thymidine in a cell of this invention as described above, an antagonist will inhibit the thymidine uptake inhibition effect caused by an androgen receptor agonist.

The disclosures of all patents, applications, publications and documents, for example brochures or technical bulletins, cited herein, are hereby expressly incorporated by reference in their entirety.

It is believed that one skilled in the art can, using the present description, including the examples, drawings, sequence listings and attendant claims, utilize the present invention to its fullest extent. The following Examples are to be construed as merely illustrative of the practice of the invention and not limitative of the remainder of the disclosure in any manner whatsoever.

### EXAMPLES

EXAMPLE 1 provides the preparation of a mammalian cell line overexpressing human androgen receptor. EXAMPLE 2 exemplifies the effect of androgens on cell proliferation of a myoblast line overexpressing androgen receptor in a [³H]-thymidine uptake assay. EXAMPLE 3 exemplifies the effect of androgen on myogenic differentiation in androgen receptor overexpressing cells according to changes in cell cycle. EXAMPLE 4 exemplifies the effect of androgen on myotube formation. EXAMPLE 5 exemplifies the effect of androgen on protein accumulation during in vitro myogenesis. EXAMPLE 6 exemplifies the effect of androgen on IGF-II and IGFBP4 gene expression in androgen receptor overexpressing myoblasts. EXAMPLE 7 exemplifies the effect of androgen treatment on apoptosis.

### EXAMPLE 1

### Mammalian Cells Overexpressing The Human Androgen Receptor

### A. Preparation of expression vector

A plasmid enabling expression of the human androgen receptor in mammalian cells was generated by standard recombinant DNA cloning techniques. A DNA fragment containing the 2577-bp human androgen receptor coding sequence for the human androgen receptor (Genbank Accession No. M23263) was ligated into the unique Pvull site of the pBl-EGFP Tet vector (Clontech, Cat. #6154-1, Palo Alto, CA). The resulting expression vector was transformed and amplified in *E. coli* strain DH5α (Life Technologies). A resulting clone was selected and found to contain the complete human androgen receptor coding sequence in the appropriate site and orientation for expression, based on restriction mapping and DNA sequencing of regions encompassing the cloning junctions.

### B. Preparation of cells

All plasmid DNA including the human androgen receptor containing expression vector was introduced into the mouse muscle-derived C2C12 cells (Yaffe and Saxel (1977); ATCC No. CRL-1772) using the transfection reagent, Lipofectamine™ (Life Technologies) according the manufacturer's recommended methods and conditions. C2C12 cells were initially transfected with the Tet-On^{TM} vector (Clontech, Palo Alto, CA) according to the manufacturer's instructions using Lipofectamine™ to generate a cell line expressing the reverse tetracycline-controlled transactivator protein. The resulting cells were propagated in a high mitogen growth medium consisting of Dulbecco's Modified Eagle's Medium containing 1.5 g/L glucose (DMEM-LG, Life Technologies) supplemented with 15% fetal bovine serum (Gemini Bio-Products) and 10 pM basic fibroblast growth factor (Collaborative Biomedical Products). Several of the resulting clones (having resistance to the antibiotic G418) were evaluated for their ability to regulated expression of a TRE2-Luc reporter gene (Clontech, Palo Alto, CA) in a tetracycline-dependent manner. One highly responsive clone was subjected to a second round of transfection with the human androgen receptor containing expression vector plus pTK-hyg as the selection vector (Clontech, Palo Alto, CA). The resulting transfected pool of cells was selected for growth in the presence of 414-517 U/ml hygromycin B (Calbiochem).

### C. Confirmation of expression of human androgen receptor

The cell line designated "C2hAR57" was identified by screening the hygromycin-resistant pool for a clones with elevated levels of human androgen receptor mRNA by Northern blot analysis. Total RNA was isolated from 50-70% confluent cultures using Trizol® (Life Technologies) according to the manufacturer's protocol. Samples containing 20 micrograms of total RNA were separated by electrophoresis on formaldehyde-containing agarose gels, transferred to a Duralon-UV™ nylon membrane (Stratagene, La Jolla, CA) by capillary blotting in 5x SSPE and hybridized to a 32P-labeled DNA fragment spanning the entire canine androgen receptor protein coding region (Lu, B., S. Smock, et al. (2001). Following hybridization, the blot was washed to moderate stringency in 0.3x SSC/0.1% SDS at 55°C. Androgen receptor transcripts were detected by phosphorimaging (Cyclone™ Storage Phosphor System, Packard Instrument Corporation). In Northern blot analysis, a 3.0-kb hybridizing band was detected in RNA from C2hAR57 cells but not in C2C12 cells, thus, confirming expression of the androgen receptor in C2hAR57.

### D. Confirmation of overexpression of the androgen receptor

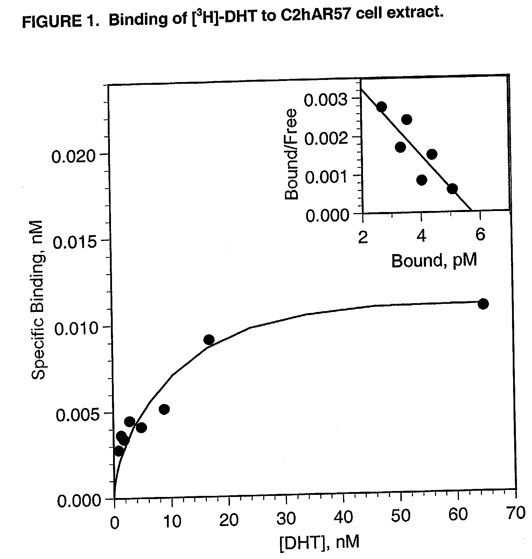

Cell extracts were prepared by sonication of C2hAR57 cells in binding buffer (50 mM Hepes pH 7.2, 1.5 mM EDTA, 5 mM DTT, 10 mM NaF,10 mM Na₂MoO₄, 10% glycerol, 0.1 mg/ml bacitracin, and 1 mM Pefabloc; 7.5 µl per cm²). The extracts were centrifuged (10,000 x g for 10 minutes at 4°C) and the resulting supernatants were frozen in liquid nitrogen and stored at -80°C. Upon thawing, the extracts were diluted with binding buffer to give 10 mg/ml total protein. Binding reactions were performed at 4°C for one hour in a volume of 0.2 ml containing 10 µl cell extract, 1nM [1,2,4,5,6,7-³H(N)]-5α-androstan-17β-ol-3-one ([³H]-DHT, 110 Curies/millimole, New England Nuclear) plus 0, 0.5, 1, 2, 4, 8, 16, 32, 64 or 2000 nM unlabeled DHT. Androgen receptor-ligand complexes were allowed to form for one hour at 4°C, then captured on hydroxylapatite-containing 96-well plate format filters (Multiscreen, Millipore, Bedford, MA). Plates were washed three times with binding buffer and bound radioactivity was quantitated by scintillation counting. The results in Table I and FIGURE 1 show that the binding of [³H]-DHT to C2hAR extract was specific. Saturable Scatchard analysis of the binding data, as shown in FIGURE 1 inset, was consistent with a single class of binding sites and receptor abundance of 11.4 femtomoles per milligram protein with a dissociation constant of 1.15 nM (K_{d} =-1/slope). For a description of Scatchard plot analysis, *see* Hulme, E. C. e. (1992) at p.69. In contrast, specific binding of [³H]-DHT to cell extract from untransfected C2C12 cells could not be demonstrated and is therefore assumed to be less than 2.2 femtomoles androgen receptor per mg protein, the lower limit of detection by this assay.

**Table I.**

| **Binding of [**^{**3**}**H]-DHT to C2hAR extract.** | | | |
|---|---|---|---|
| Total DHT nM | Specific binding pM | Free DHT pM | Bound/Free |
| 1.0 | 2.73 | 997.3 | 2.74E-3 |
| 1.5 | 3.58 | 1,496.4 | 2.39E-3 |
| 2.0 | 3.35 | 1,996.7 | 1.68E-3 |
| 3.0 | 4.42 | 2,995.6 | 1.48E-3 |
| 5.0 | 4.06 | 4,995.9 | 8.12E-4 |
| 9.0 | 5.10 | 8,994.9 | 5.66E-4 |
| 17.0 | 9.05 | 16,990 | 5.33E-4 |
| 65.0 | 10.70 | 64,989 | 1.65E-4 |

Indirect immunofluorescence was also used to confirm androgen receptor expression. C2hAR57 cells were cultured on poly-D-lysine coated glass chamber slides (Lab-Tek, Nunc, Naperville, IL), treated for 2 hours with 100 nM DHT, then rinsed with phosphate buffered saline and fixed using 2% paraformalydehyde in 0.1 M sodium phosphate, pH 7.4, for 15 minutes at room temperature. The following steps were also performed at room temperature. Cells were permeabilized using 0.1% Triton X-100 for 10 minutes and then incubated for 20 minutes in blocking solution containing 5% normal donkey serum (Jackson Immunoresearch Laboratories, West Grove, PA) in Dulbecco's phosphate buffered saline with 0.1% bovine serum albumin (PBS/BSA) (Sigma-Aldrich). Polyclonal rabbit anti-androgen receptor antisera (PA1 -111, Affinity Bioreagents, Golden, CO) was incubated on the slides for 1 hour at a concentration of 4 ug/ml in blocking solution, followed by Cy3-conjugated anti-rabbit immunoglobulin G (1:5000, Jackson lmmunoResearch Laboratories), also for one hour. Immunofluorescence was visualized on an Olympus BH-2 fluorescence microscope with a rhodamine filter (see FIGURES 2B, 2D and 2F). The same fields were also photographed under phase contrast illumination (FIGURE 2A, 2C and 2E). Androgen receptor immunofluorescence was detected in a majority of C2hAR57 cells, predominantly localized to cell nuclei. Immunofluorescence was absent when the primary antibody was omitted. Very little nuclear fluorescence was visible in DHT-treated C2C12 cells using the above procedure, even when exposure time was increased.

Hence, Example 1 illustrates the preparation of mammalian skeletal muscle myoblasts that overexpress the androgen receptor.

### E. Deposit of C2hAR57 Cells

The cell line, C2hAR57, was deposited at the American Type Tissue Culture collection (ATCC), Manassas, VA 20110-2209, US, and given the Patent Deposit Designation, PTA-4126. The deposited cell line was accepted by the ATCC on March 6, 2002 for deposit under the Budapest Treaty on the International Recognition of the Deposit of Microorganisms for the purpose of Patent Procedure. The depositor was Pfizer Inc., Eastern Point Road, Groton, CT 06340, United States of America.

### EXAMPLE 2

### [³H]-Thymidine Uptake Assay Using Muscle Cells of the Invention

C2hAR57 cells prepared according to Example 1 were plated at 1000 cells per well in 96-well tissue culture plates (Costar, Corning, NY) using DMEM-LG supplemented with 15% fetal bovine serum (Gemini Bio-Products) and incubated overnight at 37°C in 5% C0₂/95% air. The next day, the medium was replaced with medium containing the test agents, DHT, oxandrolone, testosterone, stanozolol, 2-hydroxyflutamide, 17β-estradiol and progesterone. After 48 hours exposure to the test agents, medium containing 0.1 µCi [³H]-thymidine (82 Ci/mmol, Amersham, Buckinghamshire, UK) per well was added and incubation was continued for two additional hours at 37°C. The assay was terminated by aspirating the culture medium and washing with ice-cold phosphate buffered saline followed by 5% trichloroacetic acid. The cells where then lysed by applying 0.3 N NaOH (75 µl per well) followed by 0.3 N HCI (75 µl per well) to neutralize the NaOH. The radioactivity in 50-µl aliquots was determined by liquid scintillation counting in 96-well plates (Packard Instrument Corporation) using a TopCount™ Scintillation Counter (Packard Instrument Corporation). The natural androgens (testosterone and DHT) as well as synthetic androgens (oxandrolone and stanozolol) all demonstrated dose-dependent inhibitory effects on [³H]-thymidine uptake by C2hAR57 cells (see FIGURE 4). Concentrations of up to one micromolar had little effect on C2C12 cells (see FIGURE 5). The rank order potency of these four androgens on inhibition of [³H]-thymidine uptake was consistent with their known affinities for the androgen receptor (IC₅₀ are 3.5 nM, 4.4 nM, 8.6 nM, and 28.4 nM for DHT, stanozolol, testosterone and oxandrolone, respectively). In addition, the inhibition by 0.1 nM DHT could be completely reversed by concomitant addition of the androgen antagonist 2-hydroxyflutamide (see FIGURE 6).

Hence, Example 2 demonstrates the use of the cells of the invention for identifying and characterizing androgen receptor agonists and antagonists by means of changes in the level of proliferation of the cells as illustrated by changes in the level of [³H]-thymidine uptake.

### EXAMPLE 3

### Assay of Changes in Cell Cycle of Muscle Cells of the Invention

The percentage of C2hAR57 cells at each stage of the cell cycle was determined by flow cytometry after staining with propidium iodide according to Krishan, A. (1975). C2hAR57 cells prepared according to Example 1 were plated at low density in high mitogen growth medium. Following 24 hours and 48 hours of treatment with 100 nM DHT or vehicle, during which time the cells were never allowed to exceed 30% confluence, the cells were harvested by trypsinization and resuspended in 70% ethanol in phosphate buffered saline for at least two hours at-20°C. Each cell sample was pelleted by low-speed centrifugation and resuspended in 0.5 ml phosphate buffered saline containing 100 micrograms/ml ribonuclease A and 20 microgram/ml propidium iodide for 30 minutes at room temperature. Cells exhibiting normal forward/side scatter ratios were selected for analysis of their propidium iodide fluorescence, and 10⁴ events/sample were scored using a FACScan™ flow cytometer equipped with an argon laser (488 nm emission wavelength) and Modfit™ software (Becton Dickinson Immunocytometry Systems, San Jose, CA). Treatment of C2hAR57 cells for 24 hours or 48 hours with 100 nM DHT increased the G0-G1 fraction *(see* Table II below). A concomitant decrease in the S and G2-M fractions was observed at 24 hours (p < 0.05), with a trend in the same direction after 48 hours (p < 0.1). The fraction of C2C12 cells at each stage of the cell cycle was similar to that in vehicle-treated C2hAR57 cultures and was unaffected by 100 nM DHT.

Hence, Example 3 is consistent with Example 2 in demonstrating the use of the cells of the present invention for identifying and characterizing androgen receptor agonists and antagonists by measuring changes in the level of proliferation of the cells, wherein such changes are determined according to the level of cells that are in G0-G1, G2-M and S cell cycle stage.

**Table II.**

| **Percentage of cells in defined stages of cell cycle by flow cytometric analysis of propidium iodide staining.** | | | | | | |
|---|---|---|---|---|---|---|
| **Cell Type** | **Treatment** | **Duration, h** | **N** | **G0-G1** | **G2-M** | **S** |
| C2C12 | Vehicle | 24 | 5 | 41.9 ± 0.5 | 14.9 ± 1.2 | 43.2 ± 1.1 |
| | 100 nM DHT | 24 | 5 | 40.3 ± 0.8 | 15.6 ± 1.2 | 45.3 ± 1.1 |
| | | | | | | |
| | Vehicle | 48 | 5 | 39.0 ± 0.5 | 16.6 ± 0.7 | 44.2 ± 0.5 |
| | 100 nM DHT | 48 | 5 | 38.0 ± 0.6 | 15.8 ± 0.7 | 46.2 ± 0.7 |
| | | | | | | |
| C2hAR57 | Vehicle | 24 | 5 | 40.2 ± 1.0 | 17.6 ± 0.7 | 42.2 ± 0.6 |
| | 100 nM DHT | 24 | 5 | 54.5 ± 0.7^{***} | 14.3 ± 0.4^{***} | 31.2 ± 0.6^{***} |
| | | | | | | |
| | Vehicle | 48 | 5 | 38.1 ± 1.1 | 18.9 ± 1.1 | 43.0 ± 2.1 |
| | 100 nM DHT | 48 | 4 | 51.2 ± 2.8^{**} | 15.1 ± 1.1^{*} | 33.7 ± 3.2^{*} |
| Values are mean + SEM of replicate cultures (N = 4 or 5) | | | | | | |

| | | | | | | |
|---|---|---|---|---|---|---|
| *P < 0.1, | | | | | | |
| **P < 0.05, | | | | | | |
| *** P < 0.005 versus Vehicle. | | | | | | |

### EXAMPLE 4

### Assay of Myotube Formation In Muscle Cells of the Invention

C2hAR57 cells prepared according to Example 1 were allowed to reach 50-70% confluence in high mitogen growth medium, and the medium was then switched to Dulbecco's Modified Eagle's Medium supplemented with 2% horse serum ("fusion medium"). Fusion medium was changed daily. C2hAR57 cells at low density in high mitogen growth medium appeared as small, adherent mononuclear cells. Within three days of the switch to fusion medium, a large proportion of the mononucleated myoblasts fused to form elongated, multimucleated myotubes. Myotubes formed more rapidly in cultures of C2hAr57 exposed to 100 nM DHT than those cultured in the absence of exogenous androgens. This is readily observed in cultures after 1, 2 or 3 days in fusion medium as shown in FIGURE 3. In contrast, neither the rate nor the extent of myotube formation in cultures of C2C12 was visibly affected by addition of DHT.

Hence, Example 4 demonstrates the use of the cells of the invention for identifying and characterizing androgen receptor agonists and antagonists by means of changes in the level of differentiation of the cells as illustrated by changes in the rate of myotube formation.

### EXAMPLE 5

### Assay of Protein Content In Differentiating Myotubes

*Procedure A - Three days in fusion medium.* C2hAR57 cells were plated in 12-well tissue culture plates (Costar, Corning, NY) at 4 x 10⁴ cells/well in high mitogen growth medium. After 24 hours, the medium was replaced with steroid-free growth medium (DMEM with 15% charcoal-dextran treated fetal calf serum plus 10 pM basic fibrobast growth factor). After 38 hours, vehicle (0.1% dimethylsulfoxide) or DHT (10⁻¹²-10⁻⁶ M) was added. After at least 48 hours, when cultures had reached greater than 50% confluence, the medium was switched to fusion medium with the same concentration of DHT as before. Cell extracts were prepared from cultures after three days in fusion medium by lysis in binding buffer (0.125 ml/cm²), sonication and centrifugation as described in Example 2 above. Protein concentration was determined in the supernatant fractions by the Coomassie Blue dye-binding method (Bradford, M. (1976) *Anal Biochem* 72, 248-54). As shown in Table III, there was a dose-related increase in total protein content of up to 71% in differentiating C2hAR57 cultures that had been treated with DHT, whereas there was no consistent change in similarly treated C2C12 cultures. Overall the lower protein content in C2hAR57 cultures reflects the lower density of these cultures at the time they were switched into fusion medium.

**Table III.**

| **Protein content in micrograms per well after 3 days in fusion medium (Procedure A).** | | |
|---|---|---|
| [DHT], nM | C2C12 protein, micrograms/well | C2hAR57 protein, micrograms/well |
| Vehicle | 238 ± 4 | 124 ± 4 |
| 0.001 | 245 ± 5 (+3%) | 123 ± 6 (-1%) |
| 0.01 | 228 ± 3 (-4%) | 127 ± 11 (+3%) |
| 0.1 | 246 ± 4 (+3%) | 152 ± 2 (+23%)^{*} |
| 1 | 256 ± 9 (+7%) | 188 ± 4 (+52%)^{*} |
| 10 | 243 ± 6 (+2%) ^{*} | 199 ± 10 (+61%)^{*} |
| 100 | 243 ± 6 (+2%) | 200 ± 4 (+62%)^{*} |
| 1000 | 278 ± 2 (+17%)^{*} | 212 ± 3 (+71%)^{*} |
| Value in parentheses is the percentage difference from vehicle. | | |

| | | |
|---|---|---|
| *P<0.05 versus Vehicle. | | |

*Procedure B - 24 Hours in fusion medium.* C2hAR57 cells were cultured at low density in high mitogen growth medium containing complete serum plus vehicle (0.1% dimethylsulfoxide) or DHT (10⁻¹²-10⁻⁶ M) for 48h, then trypsinized and replated at 5 x 10⁵ cells/well in 12-well tissue culture plates with the same concentration of DHT as before. One day after replating (day zero), cell extracts were prepared from one set of cultures for protein determination while a duplicate set was switched to fusion medium with continued DHT treatment. Cell extracts were prepared from the second set of cultures after 24 hours in fusion medium (day one). The change in protein content was determined by subtracting the average protein content for each treatment on day zero from the average protein content on day one. Differentiating cultures treated with vehicle showed a net loss of protein which was ameliorated and even reversed in cultures treated with increasing concentrations of DHT, as shown in Table IV.

**Table IV.**

| **Net change in protein content of C2hAR57 cultures during 24 hours in fusion medium (Procedure B)**. | |
|---|---|
| [DHT], nM | Change in protein content, micrograms/well |
| Vehicle | -107 |
| 0.001 | -93 |
| 0.1 | -56 |
| 10 | +48 |
| 1000 | +61 |

Hence, Example 5 is consistent with Example 4 in demonstrating the use of the cells of the present invention for identifying and characterizing androgen receptor agonists and antagonists by means of changes in the level of differentiation of the cells, wherein such changes are determined according to the level of protein content in the cells.

### EXAMPLE 6

### Assay of IGF-II and IGFBP4 Gene Expression In Cells that

### Overexpress the Androgen Receptor

C2C12 and C2hAR57 cells were maintained as cultures of proliferating myoblasts for four days in high mitogen growth medium with vehicle (0.1% DMSO) or 100 nM DHT, then allowed to reach high density (>70% confluence) before switching to fusion medium. Day zero samples were harvested just prior to the induction of differentiation. Additional samples were harvested after one, two, three, or five days in fusion medium (with continued addition of vehicle or DHT). Total RNA was isolated using Trizol®, and RNA concentration was determined by UV spectroscopy. Samples containing 20 micrograms of total RNA were separated by electrophoresis on formaldehyde-containing agarose gels, transferred to Duralon-UV™ nylon membrane (Stratagene, La Jolla, CA) by capillary blotting in 5x SSPE and probed sequentially for IGFBP4, IGF-ll and 18S ribosomal RNA. DNA fragments used for probes were generated by reverse transcription polymerase chain reaction using rat skeletal muscle poly A+ RNA as template. Forward primer 5'-GGCGACGAAGCCATCCACTG-3' and reverse primer 5'-CCCGGTGCAGCTCACTCTGG-3' were used to amplify a 469-bp fragment from the coding region of rat IGFBP4. Forward primer 5'-TGTTGGTGCTTCTCATCTCTTTGG-3' and reverse primer 5'-CACAGACTGATGGTACTACATTGC-3' were used to amplify a 562-base pair fragment encompassing most of the rat IGF-II coding region *(see* 562 bp partial rat IGF-II sequence disclosed in Soares, M., D. Ishii, et al. (1985) *Nuc. Acids Res.* 13(4), 1119-1134 cloned into the Eco Rl and BamHl sites of pGEM1 (Promega Corporation, Madison, Wisconsin). Forward primer 5'-ACTTTCGATGGTAGTCGCCGTGC-3' and reverse primer 5'-ATCTGATCGTCTTCGAACCTCCGA were used to amplify a 674-bp fragment from the gene encoding 18S rRNA. Double-stranded ³²P-labeled probes were synthesized by random primer extension *(see* Feinberg, A. and B. Vogelstein (1983) and Feinberg, A. P. and B. Vogelstein (1984)). Probes were denatured by boiling and used at a concentration of 1-2 x 10⁶ dpm/ml hybridization solution (50% formamide, 6X SSC, 1% SDS, 15X Denhardt's solution, 0.1 mg/ml sheared salmon sperm DNA). Following hybridization at 42°C for at least 24 hours, the membranes were washed to moderate stringency with 0.3x SSC/0.1% SDS at 55°C. Hybridization signals were detected and quantified by phosphorimaging using a Cyclone™ Storage Phosphor System with OptiQuant™, version 3.15 software (Packard Instrument Corporation). Between successive probings, the membrane was stripped by incubation at 70°C for one to three hours in a solution consisting of 5 mM TrisHCl pH 8, 0.2 mM Na₂EDTA, 0.05% sodium pyrophosphate and 0.1X Denhardt's solution. The imaging plate was exposed for 42 hours and 90 minutes to the IGFBP4 and IGF-ll hybridizations, respectively. The 18S signal was used to normalize the hybridization signals from each of the other probes.

FIGURE 8 illustrates the results of the Northern blot analysis. DHT modified the expression of all four genes to a greater degree in C2hAR57 cells compared to C2C12 cells. DHT treatment produced a reciprocal change in levels of IGFBP4 and IGF-II RNA, reducing the abundance of 2.6-kb IGFBP4 transcripts while stimulating 4.2-kb IGF-ll transcripts. The magnitude of IGFBP4 suppression by DHT after 0-2 days in fusion medium was 75%, 86% and 70% in C2hAR57 but only 20%, 51% and 44% in C2C12 cells. At the same timepoints, DHT increased IGF-ll transcripts by 10.2X, 3.6X and 2X in DHT-treated C2hAR57, but only 0.8X, 1.5X and 1.0X in C2C12.

Hence, Example 6 is consistent with Examples 4 and 5 in demonstrating the use of the cells of the present invention for identifying and characterizing androgen receptor agonists and antagonists by means of changes in the level of differentiation of the cells, wherein such changes are determined according to the level of IGF-II and IGFBP4 expression.

### EXAMPLE 7

### Assay of Apoptosis in Muscle Cells of the Invention

Parallel cultures of C2hAR57, prepared according to Example 1, and C2C12 cells were cultured in 100-mm diameter round tissue culture dishes (Falcon, Becton-Dickinson Labware, Franklin Lakes, NJ) in high mitogen growth medium with 100 nM DHT or vehicle for four days, then switched to fusion medium (with DHT or vehicle, as before). All cultures were approximately 80% confluent at the time of mitogen withdrawal. After 24 hour in fusion medium, the medium containing nonadherent cells was transferred to centrifuge tubes. The dishes were then rinsed with phosphate buffered saline and adherent cells were collected by trypsinization. Cell pellets were recovered by centrifugation and lysed in 50 mM TrisHCl, pH 8, 100 mM Na₂EDTA, 0.5% sodium dodecylsulfate. Total DNA was isolated from both the adherent and nonadherent cell fractions by standard procedures *(see,* Blin, N. and D. Stafford (1976)). DNA yields from pools of five dishes were determined by UV spectroscopy at 260 nm. Aliquots corresponding to 10 µg DNA were analyzed by electrophoresis on a 1% agarose gel in TBE buffer and visualized by ethidium bromide fluorescence with UV transillumination.

Yields of DNA from each cell line and treatment are summarized in Table V. The fraction of DNA in the nonadherent fraction of DHT-treated C2hAR57 cultures was markedly smaller than the fraction found in vehicle-treated C2hAR57 cultures. DHT treatment did not appreciably alter the fraction of nonadherent cells in the C2C12 cell line. The DNA in each sample was assessed qualitatively by agarose gel electrophoresis as shown in FIGURE 7. DNA from the nonadherent cell fractions of both C2hAR57 cultures exhibited a clear ladder of fragments in multiples of 180 bp as is typical of cells undergoing apoptosis. Based on the relative intensity of ethidium bromide fluorescence, a larger proportion of DNA in the vehicle-treated C2hAR57 sample appeared fragmented than in the DHT-treated sample. In contrast, the DNA in all of the adherent cell fractions and in the nonadherent C2C12 cell fractions was predominantly of high molecular weight (>10 kb), with no obvious fragmentation. Thus, it appears that a percentage of C2hAR57 cells detached from the culture dish and underwent apoptosis during the 24 hour period following mitogen withdrawal, and DHT treatment reduced the incidence of apoptotic cells by approximately 75%. While a similar amount of DNA was recovered from the nonadherent fraction of C2C12 cultures, this DNA was not fragmented, indicating that the cells were not apoptotic; furthermore, there was no effect of DHT on either the yield or quality of DNA in either cell fraction.

**Table V.**

| **Total DNA recovered from cell cultures after 24 hours in fusion medium.** | | | |
|---|---|---|---|
| Cell line | Treatment | DNA, nonadherent cells µg/dish (% total) | DNA, adherent cells µg/dish |
| C2C12 | Vehicle | 12.3 (2.4%) | 496 |
| C2C12 | DHT | 14.8 (2.9%) | 504 |
| C2hAR57 | Vehicle | 8.0 (2.0%) | 400 |
| C2hAR57 | DHT | 2.6 (0.5%) | 528 |

Hence, Example 7 demonstrates the use of the cells of the invention for identifying and characterizing androgen receptor agonists and antagonists by means of changes in the level of apoptosis of the cells following mitogen withdrawal.

### REFERENCES

Arends, A.J., Wyllie, A.H. (1991) *Int. Rev. Exp. Path.* 32, 223-254
Ausubel, F.M. et al. (2001) *Current Protocols in Molecular Biology,* John Wiley & Sons, New York, NY
Benoff, S. and B. N. Nadal-Ginard (1979) *Biochemistry* 18(3), 494-500
Blau, H. M., G. K. Pavlath, et al. (1985) *Science* 230(4727), 758-766
Blin, N. and D. Stafford (1976) *Nuc. Acids Res.* 3, 2303
Bradford, M. *(1976) Anal Biochem 72,* 248-54
Chang et al. (1988) *Science* 240(4850), 324-326
Christian, C. N., et al. (1977) *Science* 196(4293), 995-998
Colbere-Garapin, F. et al. (1981) *J. Mol. Biol.* 150, 1-14
Coligan, J.E. et al. (1997) Current Protocols in Immunology, Greene Pub. Associates and Wiley-lnterscience, New York, NY
Daubas, P., A. Klarsfeld, et al. (1988) *Nucleic Acids Res.* 16(4), 1251-1271
Douglass, E. C., M. Valentine, et al. (1987) [published erratum appears in *Cytogenet Cell Genet* 1988, 47(4):following 232] *Cytogenet. Cell Genet.* 45: 148-155)
Feinberg, A. and B. Vogelstein (1983) *Anal Biochem* 132(1), 6-13
Feinberg, A. P. and B. Vogelstein (1984), *Anal Biochem* 137(1), 266-267
Florini, J., D. Ewton, et al. (1996) *Endocrine Reviews* 17(5), 481-517
Gaspar et al. (1991) *Proc. Natl. Acad. Sci. USA* 88, 8606-8610
Genbank Accession No. M23263
Genbank Accession No. M23264
Genbank Accession No. M34233
Genbank Accession No. M37890
Genbank Accession No. JO5454
Hampton, R. et al. (1990) Serological Methods, a Laboratory Manual, APS Press, St. Paul MN, Sect. IV
Hartman, S.C., et al. (1988) *Proc. Natl. Acad. Sci.* USA 85, 8047-8051
He et al. (1990) *Biochem. Biophys. Res. Commun.* 171(2), 697-704
Hofbauer, et al. (2000) *J*. *Clin. Invest.* 106, 1553-1560
Hofbauer, L.C., Khosla, S (1999) *Eur. J. Endocrinol.* 140, 271-286
Hulme, E. C. e. (1992) Receptor-Ligand Interactions: A Practical Approach, New York, Oxford University Press
Jackson, J.A.: Osteoporosis in men, *In:* Favus, M.J. (ed.) (1993) *Primer on the Metabolic Bone Diseases and Disorders of Mineral Metabolism,* Raven Press, New York
Kaufmann, S.H., Hengartner, M.O. (2001) *Trends in Cell Biology* 11, 526-534
Krishan, A. (1975) *J Cell* 66,188-193
Kimes, B. W. and B. L. Brandt (1976) *Exp. Cell* Res. 98, 367-381
Liao, S., et al. *(1984) J Steroid Biochem 20, 11-17*
Lim, R. W. and S. D. Hauschka (1984) *Journal of Cell Biology* 98(2), 739-747
Lowy, I. et al. (1980) *Cell* 22, *817-823*
Lu, B., S. Smock, et al. (2001) *Mol. Cell. Biochem.* 226, 129-140.
McBurney, M. W., E. M. Jones-Villeneuve, et al. (1982) *Nature* 299(5879), 165-167
McCusker, R. and D. Clemmons (1994) *Endocrinology* 134, 2095-2102
Pound, J.D. (1998) Immunochemical Protocols, Humana Press, Totowa NJ
Rhodes, C.A. (1995) *Methods Mol. Biol.* 55,121-131
Richler, C. and Yaffe, D. (1970) *Dev. Biol.* 23, 1-22
Rhodes, C.A. (1995) *Methods Mol. Biol.* 55,121-131
Sambrook, J. et al. (1989) Molecular Cloning, A Laboratory Manual, Cold Spring Harbor Press, Plainview, NY
Scharf, D. et al. (1994) *Results Probl. Cell Differ.* 20,125-162
Schubert, D., A. J. Harris, et al. (1974) *J*. *Cell Biol.* 61, 398-413
Shiba et al. (2001) *J*. *Dermatol. Sci.* 26(3):163-168
Soares, M., D. Ishii, et al. (1985) *Nuc. Acids Res.* 13(4), 1119-1134
Rosl, F. (1992) *Nuc. Acids Res.* 20(19), 5243
Tollefsen, S., R. Lajara, et al. (1989) *J Biol Chem* 264(23), 13810-13817
Tollefsen, S., J. Sadow, et al. (1989) *Proc Natl Acad Sci* USA 86, 1543-1547
Urban, R., Y. Bodenburg, et al. (1986) *Am J Physiol* 269 (Endocrinol Metab 32), E820-E825 Yaffe, D. and O. Saxel (1977) *Nature* 270, 557-61
Wigler, M. et al. (1977) *Cell* 11, 223-232; Lowy, I. et al. (1980) *Cell 22,* 817-823
Wigler, M. et al. (1980) *Proc. Natl. Acad. Sci. USA* 77, 3567-3570
Wyllie, A.H., et al. (1980) *Int. Rev. Cytol.* 68, 251-306
Yaffe, D. and O. Saxel (1977) *Nature* 270, 557-61
International Patent Application Publication WO 93/06482 (issued in the U.S. as U.S. Patent No. 5,582,977)
International Patent Application Publication WO 94/24213 (issued in the U.S. as U.S. Patent No. 5,436,134)
International Patent Application Publication WO 02/00716
U.S. Patent 5,321,130
U.S. Patent 5,410,030
U.S. Patent 5,436,134
U.S. Patent 5,437,980
U.S. Patent 5,614,620

### ABBREVIATIONS

"DHT" means dihydrotestosterone
"DNA" means deoxyribonucleic acid
"cDNA" means complimentary DNA
"nm" means nanometer
"nM" means nanomolar
"RNA" means ribonucleaic acid
"mRNA" means messenger RNA
"UV" means ultraviolet
"SDS" means sodium dodecyl sulfate
"SSC" means saline saline citrate

## Claims

1. A mammalian skeletal myoblast that overexpresses an androgen receptor wherein said androgen receptor is a polypeptide sequence that is encoded by a polynucleotide sequence that hybridizes under high stringency conditions to a polynucleotide sequence selected from SEQ ID NO: 1 and SEQ ID NO: 2.

2. A myoblast of claim 1 wherein said androgen receptor is a polypeptide sequence that is encoded by a polynucleotide sequence selected from SEQ ID NO: 1 and SEQ ID NO: 2.

3. A transformed mammalian skeletal myoblast comprising an inserted polynucleotide sequence wherein said inserted polynucleotide sequence hybridizes under high stringency conditions to a polynucleotide sequence selected from SEQ ID NO: 1 and SEQ ID NO: 2.

4. A myoblast of claim 3 wherein said inserted polynucleotide sequence is selected from SEQ ID NO: 1 and SEQ ID NO: 2.

5. A myoblast of any one of claims 1 to 4 wherein the myoblast is a murine skeletal myoblast.

6. A C2hAR57 cell.

7. A screening method comprising:
treating a cell selected from a cell of any one of claim 1-6, with an agent that modulates the androgen receptor; and
determining the effect of said agent on said cell.

8. A screening method comprising:
treating a cell selected from a cell of any one of claim 1-6, with an agent that modulates the androgen receptor; and
determining the effect of said agent on:
the continued proliferation of said cell;
the differentiation of said cell;
the level of thymidine uptake of said cell;
whether said cell is in G0 or G1 cell cycle phase;
whether said cell is in G2-M or S cell cycle phase;
the formation of myotubes of said cell;
the level of protein content of said cell;
the level of IGF-II expression of said cell; or
the level of IGFBP4 expression of said cell.

9. A screening method for identifying an androgen receptor agonist comprising:
treating a cell of any one of claim 1-6 with a test agent;
determining the effect of said agent on the proliferation of said cell; and
characterizing the test agent as one of the following:
an androgen receptor agonist, provided the agent inhibits or terminates proliferation of said cell; or
an agent that is not androgen receptor agonist, provided the agent does not inhibit or terminate proliferation of said cell.

10. A screening method of claim 9 wherein the inhibition or termination of proliferation is determined by a method comprising measuring thymidine uptake of said cell.

11. A screening method for identifying an androgen receptor agonist comprising:
treating a cell of any one of claim 1-6 with a test agent;
determining the effect of said agent on the differentiation of said cell; and
characterizing the test agent as one of the following:
an androgen receptor agonist, provided the agent promotes the differentiation of said cell; or
an agent that is not an androgen receptor agonist, provided the agent does not promote the differentiation of said cell.

12. A screening method for identifying an androgen receptor agonist comprising:
treating a cell of any one of claim 1-6 with a test agent and mitogen; withdrawing said mitogen
determining the effect of said agent on apoptosis as a result of said mitogen withdrawal; and
characterizing the test agent as one of the following:
an androgen receptor agonist, provided the agent inhibits or prevents apoptosis of said cell; or
an agent that is not an androgen receptor agonist, provided the agent does not inhibit or prevent apoptosis of said cell.

13. A screening method for identifying an androgen receptor antagonist comprising:
treating a cell of any one of claim 1-6 with an androgen receptor agonist and a test agent;
determining the effect of said agent on:
the continued proliferation of said cell;
the differentiation of said cell;
the level of thymidine uptake of said cell;
whether the cell is in G0 or G1 cell cycle phase;
whether the cell is in G2-M or S cell cycle phase;
the formation of myotubes of said cell;
the level of protein content of said cell;
the level of IGF-ll expression of said cell; or
the level of IGFBP4 expression of said cell.

14. A screening method for identifying an androgen receptor antagonist comprising:
treating a cell of any one of claim 1-6 with mitogen, an androgen receptor agonist and a test agent;
withdrawing said mitogen
determining the effect of said agent on apoptosis of the cell.


SEQUENCE LISTING

<110>  Pfizer Products, Inc.

<120>  ANDROGEN RECEPTOR OVEREXPRESSING SKELETAL MYOBLASTS

<130>  PC11038A

<150>  60/360,655
<151>  2002-02-28

<160>  4

<170>  PatentIn version 3.1

<210>  1
<211>  3715
<212>  DNA
<213>  Homo sapiens

<400>  1
gaattccggc ggagagaacc ctctgttttc ccccactctc tctccacctc ctcctgcctt      60

ccccaccccg agtgcggagc agagatcaaa agatgaaaag gcagtcaggt cttcagtagc     120

caaaaaacaa aacaaacaaa aacaaaaaag ccgaaataaa agaaaaagat aataactcag     180

ttcttatttg cacctacttc agtggacact gaatttggaa ggtggaggat tttgtttttt     240

tcttttaaga tctgggcatc ttttgaatct acccttcaag tattaagaga cagactgtga     300

gcctagcagg gcagatcttg tccaccgtgt gtcttcttct gcacgagact ttgaggctgt     360

cagagcgctt tttgcgtggt tgctcccgca agtttccttc tctggagctt cccgcaggtg     420

ggcagctagc tgcagcgact accgcatcat cacagcctgt tgaactcttc tgagcaagag     480

aaggggaggc ggggtaaggg aagtaggtgg aagattcagc caagctcaag gatggaagtg     540

cagttagggc tgggaagggt ctaccctcgg ccgccgtcca agacctaccg aggagctttc     600

cagaatctgt tccagagcgt gcgcgaagtg atccagaacc cgggccccag gcacccagag     660

gccgcgagcg cagcacctcc cggcgccagt ttgctgctgc tgcagcagca gcagcagcag     720

cagcagcagc agcagcagca gcagcagcag caagagacta gccccaggca gcagcagcag     780

cagcagggtg aggatggttc tccccaagcc catcgtagag gccccacagg ctacctggtc     840

ctggatgagg aacagcaacc ttcacagccg cagtcggccc tggagtgcca ccccgagaga     900

ggttgcgtcc cagagcctgg agccgccgtg gccgccagca aggggctgcc gcagcagctg     960

ccagcacctc cggacgagga tgactcagct gccccatcca cgttgtccct gctgggcccc    1020

actttccccg gcttaagcag ctgctccgct gaccttaaag acatcctgag cgaggccagc    1080

accatgcaac tccttcagca acagcagcag gaagcagtat ccgaaggcag cagcagcggg    1140

agagcgaggg aggcctcggg ggctcccact tcctccaagg acaattactt agggggcact    1200

tcgaccattt ctgacaacgc caaggagttg tgtaaggcag tgtcggtgtc catgggcctg 1260

ggtgtggagg cgttggagca tctgagtcca ggggaacagc ttcgggggga ttgcatgtac 1320

gccccacttt tgggagttcc acccgctgtg cgtcccactc cttgtgcccc attggccgaa 1380

tgcaaaggtt ctctgctaga cgacagcgca ggcaagagca ctgaagatac tgctgagtat 1440

tcccctttca agggaggtta caccaaaggg ctagaaggcg agagcctagg ctgctctggc 1500

agcgctgcag cagggagctc cgggacactt gaactgccgt ctaccctgtc tctctacaag 1560

tccggagcac tggacgaggc agctgcgtac cagagtcgcg actactacaa ctttccactg 1620

gctctggccg gaccgccgcc ccctccgccg cctccccatc cccacgctcg catcaagctg 1680

gagaacccgc tggactacgg cagcgcctgg gcggctgcgg cggcgcagtg ccgctatggg 1740

gacctggcga gcctgcatgg cgcgggtgca gcgggacccg gttctgggtc accctcagcc 1800

gccgcttcct catcctggca cactctcttc acagccgaag aaggccagtt gtatggaccg 1860

tgtggtggtg gtgggggtgg tggtggcggc ggcggcggcg gcggcggcgg cggcggcggc 1920

ggcggcggcg gcggcggcgg cggcgaggcg gaagctgtag ccccctacgg ctacactcgg 1980

ccccctcagg ggctggcggg ccaggaaagc gacttcaccg cacctgatgt gtggtaccct 2040

ggcggcatgg tgagcagagt gccctatccc agtcccactt gtgtcaaaag cgaaatgggc 2100

ccctggatgg atagctactc cggaccttac ggggacatgc gtttggagac tgccagggac 2160

catgttttgc ccattgacta ttactttcca ccccagaaga cctgcctgat ctgtggagat 2220

gaagcttctg ggtgtcacta tggagctctc acatgtggaa gctgcaaggt cttcttcaaa 2280

agagccgctg aagggaaaca gaagtacctg tgcgccagca gaaatgattg cactattgat 2340

aaattccgaa ggaaaaattg tccatcttgt cgtcttcgga aatgttatga agcagggatg 2400

actctgggag cccggaagct gaagaaactt ggtaatctga aactacagga ggaaggagag 2460

gcttccagca ccaccagccc cactgaggag acaacccaga agctgacagt gtcacacatt 2520

gaaggctatg aatgtcagcc catctttctg aatgtcctgg aagccattga gccaggtgta 2580

gtgtgtgctg gacacgacaa caaccagccc gactcctttg cagccttgct ctctagcctc 2640

aatgaactgg gagagagaca gcttgtacac gtggtcaagt gggccaaggc cttgcctggc 2700

ttccgcaact tacacgtgga cgaccagatg gctgtcattc agtactcctg gatggggctc 2760

atggtgtttg ccatgggctg gcgatccttc accaatgtca actccaggat gctctacttc 2820

gcccctgatc tggttttcaa tgagtaccgc atgcacaagt cccggatgta cagccagtgt 2880

gtccgaatga ggcacctctc tcaagagttt ggatggctcc aaatcacccc ccaggaattc 2940

ctgtgcatga aagcactgct actcttcagc attattccag tggatgggct gaaaaatcaa 3000

aaattctttg atgaacttcg aatgaactac atcaaggaac tcgatcgtat cattgcatgc 3060

aaaagaaaaa atcccacatc ctgctcaaga cgcttctacc agctcaccaa gctcctggac 3120

tccgtgcagc ctattgcgag agagctgcat cagttcactt ttgacctgct aatcaagtca 3180

cacatggtga gcgtggactt tccggaaatg atggcagaga tcatctctgt gcaagtgccc 3240

aagatccttt ctgggaaagt caagcccatc tatttccaca cccagtgaag cattggaaac 3300

cctatttccc caccccagct catgccccct ttcagatgtc ttctgcctgt tataactctg 3360

cactactcct ctgcagtgcc ttgtttaatt tcctctattg atgtacagtc tgtcatggaa 3420

ttctatttgc tgggcttttt ttttctcttt ctctcctttc tttttcttct tccctcccta 3480

tctaaccctc ccatggcacc ttcagacttt gcttcccatt gtggctccta tctgtgtttt 3540

gaatggtgtt gtatgccttt aaatctgtga tgatcctcat atggcccagt gtcaagttgt 3600

gcttgtttac agcactactc tgtgccagcc acacaaacgt ttacttatct tatgccacgg 3660

gaagtttaga gagctaagat tatctgggga aatcaaaaca aaaacacccg aattc 3715

<210> 2
<211> 3231
<212> DNA
<213> Homo sapiens

<400> 2
agctagctgc agcgactacc gcatcatcac agcctgttga actcttctga gcaagagaag 60

gggaggcggg gtaagggaag taggtggaag attcagccaa gctcaaggat ggaagtgcag 120

ttagggctgg gaagggtcta ccctcggccg ccgtccaaga cctaccgagg agctttccag 180

aatctgttcc agagcgtccg cgaagtgatc cagaacccgg gccccaggca cccagaggcc 240

gcgagcgcag cacctcccgg cgccagtttg ctgctgctgc agcagcagca gcagcagcag 300

cagcagcagc agcagcagca gcagcaagag actagcccca ggcagcagca gcagcagcag 360

ggtgaggatg gttctcccca agcccatcgt agaggcccca caggctacct ggtcctggat 420

gaggaacagc aaccttcaca gccgcagtcg gccctggagt gccaccccga gagaggttgc 480

gtcccagagc ctggagccgc cgtggccgcc agcaaggggc tgccgcagca gctgccagca 540

cctccggacg aggatgactc agctgcccca tccacgttgt ccctgctggc ccccactttc 600

cccggcttaa gcagctgctc cgctgacctt aaagacatcc tgagcgaggc cagcaccatg 660

caactccttc agcaacagca gcaggaagca gtatccgaag gcagcagcag cgggagagcg 720

agggaggcct cgggggctcc cacttcctcc aaggacaatt acttaggggg cacttcgacc 780

atttctgaca acgccaagga gttgtgtaag gcagtgtcgg tgtccatggg cctgggtgtg 840

gaggcgttgg agcatctgag tccaggggaa cagcttcggg gggattgcat gtacgcccca 900

cttttgggag ttccacccgc tgtgcgtccc actccttgtg ccccattggc cgaatgcaaa 960

ggttctctgc tagacgacag cgcaggcaag agcactgaag atactgctga gtattcccct 1020

ttcaagggag gttacaccaa agggctagaa ggcgagagcc taggctgctc tggcagcgct 1080

gcagcaggga gctccgggac acttgaactg ccgtctaccc tgtctctcta caagtccgga 1140

gcactggacg aggcagctgc gtaccagagt cgcgactact acaactttcc actggctctg 1200

gccggaccgc cgccccctcc gccgcctccc catccccacg ctcgcatcaa gctggagaac 1260

ccgctggact acggcagcgc ctgggcggct gcggcggcgc agtgccgcta tggggacctg 1320

gcgagcctgc atggcgcggg tgcagcggga cccggttctg ggtcaccctc agccgccgct 1380

tcctcatcct ggcacactct cttcacagcc gaagaaggcc agttgtatgg accgtgtggt 1440

ggtggtgggg gtggtggcgg cggcggcggc ggcggcggcg gcggcgaggc gggagctgta 1500

gccccctacg gctacactcg gccccctcag gggctggcgg gccaggaaag cgacttcacc 1560

gcacctgatg tgtggtaccc tggcggcatg gtgagcagag tgccctatcc cagtcccact 1620

tgtgtcaaaa gcgaaatggg cccctggatg gatagctact ccggacctta cggggacatg 1680

cgtttggaga ctgccaggga ccatgttttg cccattgact attactttcc accccagaag 1740

acctgcctga tctgtggaga tgaagcttct gggtgtcact atggagctct cacatgtgga 1800

agctgcaagg tcttcttcaa aagagccgct gaagggaaac agaagtacct gtgcgccagc 1860

agaaatgatt gcactattga taaattccga aggaaaaatt gtccatcttg tcgtcttcgg 1920

aaatgttatg aagcagggat gactctggga gcccggaagc tgaagaaact tggtaatctg 1980

aaactacagg aggaaggaga ggcttccagc accaccagcc ccactgagga gacaacccag 2040

aagctgacag tgtcacacat tgaaggctat gaatgtcagc ccatctttct gaatgtcctg 2100

gaagccattg agccaggtgt agtgtgtgct ggacacgaca acaaccagcc cgactccttt 2160

gcagccttgc tctctagcct caatgaactg ggagagagac agcttgtaca cgtggtcaag 2220

tgggccaagg ccttgcctgg cctccgcaac ttacacgtgg acgaccagat ggctgtcatt 2280

cagtactcct ggatggggct catggtgttt gccatgggct ggcgatcctt caccaatgtc 2340

aactccagga tgctctactt cgcccctgat ctggttttca atgagtaccg catgcacaag 2400

tcccggatgt acagccagtg tgtccgaatg aggcacctct ctcaagagtt tggatggctc 2460

caaatcaccc cccaggaatt cctgtgcatg aaagccatgc tactcttcag cattattcca 2520

gtggatgggc tgaaaaatca aaaattcttt gatgaacttc gaatgaacta catcaaggaa 2580

ctcgatcgta tcattgcatg caaaagaaaa aatcccacat cctgctcaag acgcttctac 2640

cagctcacca agctcctgga ctccgtgcag cctattgcga gagagctgca tcagttcact 2700

tttgacctgc taatcaagtc acacatggtg agcgtggact ttccggaaat gatggcagag 2760

atcatctctg tgcaagtgcc caagatcctt tctgggaaag tcaagcccat ctatttccac 2820

acccagtgaa gcattggaaa ccctatttcc ccaccccagc tcatgccccc tttcagatgt 2880

cttctgcctg ttataactct gcactactcc tctgcagtgc cttggggaat ttcctctatt 2940

gatgtacagt ctgtcatgaa catgttcctg aattctatct gctgggcttt ttttttctct 3000

ttctctcctt tctttttctt cttccctccc tatctaaccc tcccatggca ccttcagact 3060

ttgcttccca ttgtggctcc tatctgtgtt ttgaatggtg ttgtatgcct taaatctgtg 3120

atgatcctca tatggcccag tgtcaagttg tgcttgttta cacgcatctc tgtgccagcc 3180

acacaaaccg tttacttact taccgcaagg gaacttagag agctagaatt c 3231

<210> 3
<211> 918
<212> PRT
<213> Homo sapiens

<400> 3

Met Glu Val Gln Leu Gly Leu Gly Arg Val Tyr Pro Arg Pro Pro Ser
1               5                   10                  15

Lys Thr Tyr Arg Gly Ala Phe Gln Asn Leu Phe Gln Ser Val Arg Glu
            20                  25                  30

Val Ile Gln Asn Pro Gly Pro Arg His Pro Glu Ala Ala Ser Ala Ala
        35                  40                  45

Pro Pro Gly Ala Ser Leu Leu Leu Leu Gln Gln Gln Gln Gln Gln Gln
    50                  55                  60

Gln Gln Gln Gln Gln Gln Gln Gln Gln Gln Glu Thr Ser Pro Arg Gln
65                  70                  75                  80

Gln Gln Gln Gln Gln Gly Glu Asp Gly Ser Pro Gln Ala His Arg Arg
                85                  90                  95

Gly Pro Thr Gly Tyr Leu Val Leu Asp Glu Glu Gln Gln Pro Ser Gln
            100                 105                 110

Pro Gln Ser Ala Leu Glu Cys His Pro Glu Arg Gly Cys Val Pro Glu
        115                 120                 125

Pro Gly Ala Ala Val Ala Ala Ser Lys Gly Leu Pro Gln Gln Leu Pro
    130                 135                 140

Ala Pro Pro Asp Glu Asp Asp Ser Ala Ala Pro Ser Thr Leu Ser Leu
145                 150                 155                 160

Leu Gly Pro Thr Phe Pro Gly Leu Ser Ser Cys Ser Ala Asp Leu Lys
                165                 170                 175

Asp Ile Leu Ser Glu Ala Ser Thr Met Gln Leu Leu Gln Gln Gln Gln
            180                 185                 190

Gln Glu Ala Val Ser Glu Gly Ser Ser Ser Gly Arg Ala Arg Glu Ala
        195                 200                 205

Ser Gly Ala Pro Thr Ser Ser Lys Asp Asn Tyr Leu Gly Gly Thr Ser
    210                 215                 220

Thr Ile Ser Asp Asn Ala Lys Glu Leu Cys Lys Ala Val Ser Val Ser
225                 230                 235                 240

Met Gly Leu Gly Val Glu Ala Leu Glu His Leu Ser Pro Gly Glu Gln
                245                 250                 255

Leu Arg Gly Asp Cys Met Tyr Ala Pro Leu Leu Gly Val Pro Pro Ala
            260                 265                 270

Val Arg Pro Thr Pro Cys Ala Pro Leu Ala Glu Cys Lys Gly Ser Leu
        275                 280                 285

Leu Asp Asp Ser Ala Gly Lys Ser Thr Glu Asp Thr Ala Glu Tyr Ser
    290                 295                 300

Pro Phe Lys Gly Gly Tyr Thr Lys Gly Leu Glu Gly Glu Ser Leu Gly
305                 310                 315                 320

Cys Ser Gly Ser Ala Ala Ala Gly Ser Ser Gly Thr Leu Glu Leu Pro
                325                 330                 335

Ser Thr Leu Ser Leu Tyr Lys Ser Gly Ala Leu Asp Glu Ala Ala Ala
            340                 345                 350

Tyr Gln Ser Arg Asp Tyr Tyr Asn Phe Pro Leu Ala Leu Ala Gly Pro
        355                 360                 365

Pro Pro Pro Pro Pro Pro Pro His Pro His Ala Arg Ile Lys Leu Glu
    370                 375                 380

Asn Pro Leu Asp Tyr Gly Ser Ala Trp Ala Ala Ala Ala Ala Gln Cys
385                 390                 395                 400

Arg Tyr Gly Asp Leu Ala Ser Leu His Gly Ala Gly Ala Ala Gly Pro

                405                 410                 415

Gly Ser Gly Ser Pro Ser Ala Ala Ala Ser Ser Ser Trp His Thr Leu
            420                 425                 430

Phe Thr Ala Glu Glu Gly Gln Leu Tyr Gly Pro Cys Gly Gly Gly Gly
        435                 440                 445

Gly Gly Gly Gly Gly Gly Gly Gly Gly Gly Gly Gly Gly Gly Gly Gly
    450                 455                 460

Gly Gly Gly Gly Gly Gly Gly Glu Ala Glu Ala Val Ala Pro Tyr Gly
465                 470                 475                 480

Tyr Thr Arg Pro Pro Gln Gly Leu Ala Gly Gln Glu Ser Asp Phe Thr
                485                 490                 495

Ala Pro Asp Val Trp Tyr Pro Gly Gly Met Val Ser Arg Val Pro Tyr
            500                 505                 510

Pro Ser Pro Thr Cys Val Lys Ser Glu Met Gly Pro Trp Met Asp Ser
        515                 520                 525

Tyr Ser Gly Pro Tyr Gly Asp Met Arg Leu Glu Thr Ala Arg Asp His
    530                 535                 540

Val Leu Pro Ile Asp Tyr Tyr Phe Pro Pro Gln Lys Thr Cys Leu Ile
545                 550                 555                 560

Cys Gly Asp Glu Ala Ser Gly Cys His Tyr Gly Ala Leu Thr Cys Gly
                565                 570                 575

Ser Cys Lys Val Phe Phe Lys Arg Ala Ala Glu Gly Lys Gln Lys Tyr
            580                 585                 590

Leu Cys Ala Ser Arg Asn Asp Cys Thr Ile Asp Lys Phe Arg Arg Lys
        595                 600                 605

Asn Cys Pro Ser Cys Arg Leu Arg Lys Cys Tyr Glu Ala Gly Met Thr
    610                 615                 620

Leu Gly Ala Arg Lys Leu Lys Lys Leu Gly Asn Leu Lys Leu Gln Glu
625                 630                 635                 640

Glu Gly Glu Ala Ser Ser Thr Thr Ser Pro Thr Glu Glu Thr Thr Gln
                645                 650                 655

Lys Leu Thr Val Ser His Ile Glu Gly Tyr Glu Cys Gln Pro Ile Phe
            660                 665                 670

Leu Asn Val Leu Glu Ala Ile Glu Pro Gly Val Val Cys Ala Gly His
        675                 680                 685

Asp Asn Asn Gln Pro Asp Ser Phe Ala Ala Leu Leu Ser Ser Leu Asn
    690                 695                 700

Glu Leu Gly Glu Arg Gln Leu Val His Val Val Lys Trp Ala Lys Ala
705                 710                 715                 720

Leu Pro Gly Phe Arg Asn Leu His Val Asp Asp Gln Met Ala Val Ile
                725                 730                 735

Gln Tyr Ser Trp Met Gly Leu Met Val Phe Ala Met Gly Trp Arg Ser
            740                 745                 750

Phe Thr Asn Val Asn Ser Arg Met Leu Tyr Phe Ala Pro Asp Leu Val
        755                 760                 765

Phe Asn Glu Tyr Arg Met His Lys Ser Arg Met Tyr Ser Gln Cys Val
    770                 775                 780

Arg Met Arg His Leu Ser Gln Glu Phe Gly Trp Leu Gln Ile Thr Pro
785                 790                 795                 800

Gln Glu Phe Leu Cys Met Lys Ala Leu Leu Leu Phe Ser Ile Ile Pro
                805                 810                 815

Val Asp Gly Leu Lys Asn Gln Lys Phe Phe Asp Glu Leu Arg Met Asn
            820                 825                 830

Tyr Ile Lys Glu Leu Asp Arg Ile Ile Ala Cys Lys Arg Lys Asn Pro
        835                 840                 845

Thr Ser Cys Ser Arg Arg Phe Tyr Gln Leu Thr Lys Leu Leu Asp Ser
    850                 855                 860

Val Gln Pro Ile Ala Arg Glu Leu His Gln Phe Thr Phe Asp Leu Leu
865                 870                 875                 880

Ile Lys Ser His Met Val Ser Val Asp Phe Pro Glu Met Met Ala Glu
                885                 890                 895

Ile Ile Ser Val Gln Val Pro Lys Ile Leu Ser Gly Lys Val Lys Pro

900                 905                 910

Ile Tyr Phe His Thr Gln
        915

<210>  4
<211>  906
<212>  PRT
<213>  Homo sapiens

<400>  4
   Met Glu Val Gln Leu Gly Leu Gly Arg Val Tyr Pro Arg Pro Pro Ser
   1               5                   10                  15
   Lys Thr Tyr Arg Gly Ala Phe Gln Asn Leu Phe Gln Ser Val Arg Glu
               20                  25                  30
   Val Ile Gln Asn Pro Gly Pro Arg His Pro Glu Ala Ala Ser Ala Ala
           35                  40                  45
   Pro Pro Gly Ala Ser Leu Leu Leu Leu Gln Gln Gln Gln Gln Gln Gln
       50                  55                  60
   Gln Gln Gln Gln Gln Gln Gln Gln Gln Glu Thr Ser Pro Arg Gln Gln
   65                  70                  75                  80
   Gln Gln Gln Gln Gly Glu Asp Gly Ser Pro Gln Ala His Arg Arg Gly
                   85                  90                  95
   Pro Thr Gly Tyr Leu Val Leu Asp Glu Glu Gln Gln Pro Ser Gln Pro
               100                 105                 110
   Gln Ser Ala Leu Glu Cys His Pro Glu Arg Gly Cys Val Pro Glu Pro
           115                 120                 125
   Gly Ala Ala Val Ala Ala Ser Lys Gly Leu Pro Gln Gln Leu Pro Ala
       130                 135                 140
   Pro Pro Asp Glu Asp Asp Ser Ala Ala Pro Ser Thr Leu Ser Leu Leu
   145                 150                 155                 160
   Ala Pro Thr Phe Pro Gly Leu Ser Ser Cys Ser Ala Asp Leu Lys Asp
                   165                 170                 175
   Ile Leu Ser Glu Ala Ser Thr Met Gln Leu Leu Gln Gln Gln Gln Gln
               180                 185                 190
   Glu Ala Val Ser Glu Gly Ser Ser Ser Gly Arg Ala Arg Glu Ala Ser
           195                 200                 205
   Gly Ala Pro Thr Ser Ser Lys Asp Asn Tyr Leu Gly Gly Thr Ser Thr
       210                 215                 220
   Ile Ser Asp Asn Ala Lys Glu Leu Cys Lys Ala Val Ser Val Ser Met
   225                 230                 235                 240
   Gly Leu Gly Val Glu Ala Leu Glu His Leu Ser Pro Gly Glu Gln Leu
                   245                 250                 255
   Arg Gly Asp Cys Met Tyr Ala Pro Leu Leu Gly Val Pro Pro Ala Val
               260                 265                 270
   Arg Pro Thr Pro Cys Ala Pro Leu Ala Glu Cys Lys Gly Ser Leu Leu
           275                 280                 285
   Asp Asp Ser Ala Gly Lys Ser Thr Glu Asp Thr Ala Glu Tyr Ser Pro
       290                 295                 300
   Phe Lys Gly Gly Tyr Thr Lys Gly Leu Glu Gly Glu Ser Leu Gly Cys
   305                 310                 315                 320
   Ser Gly Ser Ala Ala Ala Gly Ser Ser Gly Thr Leu Glu Leu Pro Ser
                   325                 330                 335
   Thr Leu Ser Leu Tyr Lys Ser Gly Ala Leu Asp Glu Ala Ala Ala Tyr
               340                 345                 350
   Gln Ser Arg Asp Tyr Tyr Asn Phe Pro Leu Ala Leu Ala Gly Pro Pro
           355                 360                 365
   Pro Pro Pro Pro Pro Pro His Pro His Ala Arg Ile Lys Leu Glu Asn
       370                 375                 380
   Pro Leu Asp Tyr Gly Ser Ala Trp Ala Ala Ala Ala Ala Gln Cys Arg
   385                 390                 395                 400
   Tyr Gly Asp Leu Ala Ser Leu His Gly Ala Gly Ala Ala Gly Pro Gly
                   405                 410                 415
   Ser Gly Ser Pro Ser Ala Ala Ala Ser Ser Ser Trp His Thr Leu Phe
               420                 425                 430
   Thr Ala Glu Glu Gly Gln Leu Tyr Gly Pro Cys Gly Gly Gly Gly Gly
           435                 440                 445
   Gly Gly Gly Gly Gly Gly Gly Gly Gly Gly Gly Glu Ala Gly Ala Val
       450                 455                 460
   Ala Pro Tyr Gly Tyr Thr Arg Pro Pro Gln Gly Leu Ala Gly Gln Glu
   465                 470                 475                 480
   Ser Asp Phe Thr Ala Pro Asp Val Trp Tyr Pro Gly Gly Met Val Ser
                   485                 490                 495
   Arg Val Pro Tyr Pro Ser Pro Thr Cys Val Lys Ser Glu Met Gly Pro
               500                 505                 510
   Trp Met Asp Ser Tyr Ser Gly Pro Tyr Gly Asp Met Arg Leu Glu Thr
           515                 520                 525
   Ala Arg Asp His Val Leu Pro Ile Asp Tyr Tyr Phe Pro Pro Gln Lys
       530                 535                 540
   Thr Cys Leu Ile Cys Gly Asp Glu Ala Ser Gly Cys His Tyr Gly Ala
   545                 550                 555                 560
   Leu Thr Cys Gly Ser Cys Lys Val Phe Phe Lys Arg Ala Ala Glu Gly
                   565                 570                 575
   Lys Gln Lys Tyr Leu Cys Ala Ser Arg Asn Asp Cys Thr Ile Asp Lys
               580                 585                 590
   Phe Arg Arg Lys Asn Cys Pro Ser Cys Arg Leu Arg Lys Cys Tyr Glu
           595                 600                 605
   Ala Gly Met Thr Leu Gly Ala Arg Lys Leu Lys Lys Leu Gly Asn Leu
       610                 615                 620
   Lys Leu Gln Glu Glu Gly Glu Ala Ser Ser Thr Thr Ser Pro Thr Glu
   625                 630                 635                 640
   Glu Thr Thr Gln Lys Leu Thr Val Ser His Ile Glu Gly Tyr Glu Cys
                   645                 650                 655
   Gln Pro Ile Phe Leu Asn Val Leu Glu Ala Ile Glu Pro Gly Val Val
               660                 665                 670
   Cys Ala Gly His Asp Asn Asn Gln Pro Asp Ser Phe Ala Ala Leu Leu
   675                 680                 685
   Ser Ser Leu Asn Glu Leu Gly Glu Arg Gln Leu Val His Val Val Lys
       690                 695                 700
   Trp Ala Lys Ala Leu Pro Gly Leu Arg Asn Leu His Val Asp Asp Gln
   705                 710                 715                 720
   Met Ala Val Ile Gln Tyr Ser Trp Met Gly Leu Met Val Phe Ala Met
                   725                 730                 735
   Gly Trp Arg Ser Phe Thr Asn Val Asn Ser Arg Met Leu Tyr Phe Ala
               740                 745                 750
   Pro Asp Leu Val Phe Asn Glu Tyr Arg Met His Lys Ser Arg Met Tyr
           755                 760                 765
   Ser Gln Cys Val Arg Met Arg His Leu Ser Gln Glu Phe Gly Trp Leu
       770                 775                 780
   Gln Ile Thr Pro Gln Glu Phe Leu Cys Met Lys Ala Met Leu Leu Phe
   785                 790                 795                 800
   Ser Ile Ile Pro Val Asp Gly Leu Lys Asn Gln Lys Phe Phe Asp Glu
                   805                 810                 815
   Leu Arg Met Asn Tyr Ile Lys Glu Leu Asp Arg Ile Ile Ala Cys Lys
               820                 825                 830
   Arg Lys Asn Pro Thr Ser Cys Ser Arg Arg Phe Tyr Gln Leu Thr Lys
           835                 840                 845
   Leu Leu Asp Ser Val Gln Pro Ile Ala Arg Glu Leu His Gln Phe Thr
       850                 855                 860
   Phe Asp Leu Leu Ile Lys Ser His Met Val Ser Val Asp Phe Pro Glu
   865                 870                 875                 880
   Met Met Ala Glu Ile Ile Ser Val Gln Val Pro Lys Ile Leu Ser Gly
                   885                 890                 895
   Lys Val Lys Pro Ile Tyr Phe His Thr Gln
               900                 905